(19) Europäisches Patentamt
European Patent Office
Office européen des brevets

(11) EP 2 553 160 B1

(12) EUROPEAN PATENT SPECIFICATION

(45) Date of publication and mention of the grant of the patent:
**22.04.2015 Bulletin 2015/17**

(21) Application number: **11707325.4**

(22) Date of filing: **01.03.2011**

(51) Int Cl.:
***D06M 11/50*** *(2006.01)*
***D06M 16/00*** *(2006.01)*
***C12N 9/14*** *(2006.01)*
***C12N 9/10*** *(2006.01)*
***D06M 11/82*** *(2006.01)*
***D06M 11/55*** *(2006.01)*
***D06P 5/22*** *(2006.01)*
***D06M 101/12*** *(2006.01)*

(86) International application number:
**PCT/US2011/026613**

(87) International publication number:
**WO 2011/119301 (29.09.2011 Gazette 2011/39)**

(54) **TREATMENT OF KERATINOUS FIBERS WITH AN ENZYME HAVING PERHYDROLASE ACTIVITY**

BEHANDLUNG VON KERATINFASERN MIT EINEM PERHYDROLASE-AKTIVEN ENZYM

TRAITEMENT DE FIBRES KÉRATINIQUES AU MOYEN D’UNE ENZYME À ACTIVITÉ DE PERHYDROLASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2010 US 317915 P**

(43) Date of publication of application:
**06.02.2013 Bulletin 2013/06**

(73) Proprietor: **Danisco US Inc.**
**Palo Alto, CA 94304 (US)**

(72) Inventor: **YOON, Mee-Young**
**Palo Alto, California 94304 (US)**

(74) Representative: **Casley, Christopher Stuart et al**
**Mewburn Ellis LLP**
**33 Gutter Lane**
**London**
**EC2V 8AS (GB)**

(56) References cited:
**WO-A1-2010/030769**
**US-A- 3 634 020**
**US-A1- 2003 154 555**
**US-A1- 2007 167 344**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 553 160 B1

SEQUENCE LISTING

<110> Danisco US Inc. Yoon, Mee-Young

<120> Treatment of Keratinous Fibers with an Enzyme Having Perhydrolase Activity

<130> 31505WO

<140> PCT/US2011/026613
<141> 2011-03-01

<150> US 61/317,915
<151> 2010-03-26

<160> 2

<170> PatentIn version 3.5

<210> 1
<211> 216
<212> PRT
<213> Mycobacterium smegmatis

<400> 1

```
Met Ala Lys Arg Ile Leu Cys Phe Gly Asp Ser Leu Thr Trp Gly Trp
1               5                   10                  15
Val Pro Val Glu Asp Gly Ala Pro Thr Glu Arg Phe Ala Pro Asp Val
            20                  25                  30
Arg Trp Thr Gly Val Leu Ala Gln Gln Leu Gly Ala Asp Phe Glu Val
        35                  40                  45
Ile Glu Glu Gly Leu Ser Ala Arg Thr Thr Asn Ile Asp Asp Pro Thr
    50                  55                  60
Asp Pro Arg Leu Asn Gly Ala Ser Tyr Leu Pro Ser Cys Leu Ala Thr
65                  70                  75                  80
His Leu Pro Leu Asp Leu Val Ile Ile Met Leu Gly Thr Asn Asp Thr
                85                  90                  95
Lys Ala Tyr Phe Arg Arg Thr Pro Leu Asp Ile Ala Leu Gly Met Ser
            100                 105                 110
Val Leu Val Thr Gln Val Leu Thr Ser Ala Gly Gly Val Gly Thr Thr
        115                 120                 125
Tyr Pro Ala Pro Lys Val Leu Val Val Ser Pro Pro Pro Leu Ala Pro
    130                 135                 140
Met Pro His Pro Trp Phe Gln Leu Ile Phe Glu Gly Gly Glu Gln Lys
145                 150                 155                 160
Thr Thr Glu Leu Ala Arg Val Tyr Ser Ala Leu Ala Ser Phe Met Lys
                165                 170                 175
Val Pro Phe Phe Asp Ala Gly Ser Val Ile Ser Thr Asp Gly Val Asp
            180                 185                 190
Gly Ile His Phe Thr Glu Ala Asn Asn Arg Asp Leu Gly Val Ala Leu
        195                 200                 205
Ala Glu Gln Val Arg Ser Leu Leu
    210                 215
```

<210> 2
<211> 216
<212> PRT
<213> Mycobacterium smegmatis

<400> 2

```
Met Ala Lys Arg Ile Leu Cys Phe Gly Asp Ser Leu Thr Trp Gly Trp
1               5                   10                  15
```

```
Val Pro Val Glu Asp Gly Ala Pro Thr Glu Arg Phe Ala Pro Asp Val
            20                  25                  30
Arg Trp Thr Gly Val Leu Ala Gln Gln Leu Gly Ala Asp Phe Glu Val
        35                  40                  45
Ile Glu Glu Gly Leu Val Ala Arg Thr Thr Asn Ile Asp Asp Pro Thr
    50                  55                  60
Asp Pro Arg Leu Asn Gly Ala Ser Tyr Leu Pro Ser Cys Leu Ala Thr
65                  70                  75                  80
His Leu Pro Leu Asp Leu Val Ile Ile Met Leu Gly Thr Asn Asp Thr
                85                  90                  95
Lys Ala Tyr Phe Arg Arg Thr Pro Leu Asp Ile Ala Leu Gly Met Ser
            100                 105                 110
Val Leu Val Thr Gln Val Leu Thr Ser Ala Gly Gly Val Gly Thr Thr
        115                 120                 125
Tyr Pro Ala Pro Lys Val Leu Val Val Ser Pro Pro Pro Leu Ala Pro
    130                 135                 140
Met Pro His Pro Trp Phe Gln Leu Ile Phe Glu Gly Gly Glu Gln Lys
145                 150                 155                 160
Thr Thr Glu Leu Ala Arg Val Tyr Ser Ala Leu Ala Ser Phe Met Lys
                165                 170                 175
Val Pro Phe Phe Asp Ala Gly Ser Val Ile Ser Thr Asp Gly Val Asp
            180                 185                 190
Gly Ile His Phe Thr Glu Ala Asn Asn Arg Asp Leu Gly Val Ala Leu
        195                 200                 205
Ala Glu Gln Val Arg Ser Leu Leu
    210                 215
```

## Description

### TECHNICAL FIELD

The present methods relate to the treatment of keratinous fibers and fabrics comprising such fibers with enzymatically-generated peracids under aqueous conditions. The treatment reduces felting and increases dye uptake.

### BACKGROUND

When textiles made from keratinous fibers, such as wool, are subjected to mechanical manipulation in the wet state, they have a tendency to shrink, sometime dramatically. Such shrinkage is known as "felting." Felting is generally undesirable and irreversible, and the fabric or garment is rendered unusable. Felting tendency can be reduced by chemically modifying the surface of the textiles, which changes the frictional properties of the textiles by reducing or masking the scale structure.

Chlorination is the oldest and most well known method for reducing felting and increasing dye uptake. However, chlorine-containing compounds and their breakdown products are harmful to the environment and to textile workers. Similar environmental and safety issues are associated with resin finishing, since the resin is typically crosslinked with epichlorohydrin.

The use of peracids to modify the surface of wool fibers has been described (*e.g.,* U.S. Patent No. 3,634,020); however, such methods are performed in anhydrous organic solutions. Environmental and safety issues associated with the use and storage of concentrated peracid solutions and organic solvents preclude such methods from being commercially viable.

WO 2010/030769 and US 2007/0167344 A1 describe use of a composition comprising a perhydrolase, an ester substrate and a source of hydrogen peroxide for use in textile bleaching and cleaning compositions, respectively.

US 2003/0154555 A1 discloses the use of sodium sulfite and transglutaminase in anti-felting of wool.

The need exist for safer, more environmentally friendly, and more effective compositions and methods for reducing felting and increasing the dye uptake or wool and other fabrics made from keratinous fibers.

### SUMMARY

Described are methods for chemically modifying keratinous fibers, e.g., to reduce the felting, increase the dye uptake, and/or reduce the prickling tendency, of textiles comprising such fibers.

In some aspects, a method for reducing felting of wool or another textile made from keratinous fibers, the method comprising: contacting the textile with an aqueous composition comprising an enzyme having perhydrolase activity, an ester substrate for the enzyme, and a source of hydrogen peroxide, wherein the enzyme generates peracids *in situ* in an aqueous medium, and wherein the peracids modify the textile, thereby reducing the tendency of the textile to felt. In some embodiments, the enzyme may generates peracids *in situ* prior to contacting the textile with the aqueous composition. In some embodiments, contacting the textile with the aqueous composition occurs prior to the enzyme generating peracids *in situ.* In some embodiments, contacting the textile with the aqueous composition and the enzyme generating peracids *in situ* occur simultaneously.

In a particular aspect, a method for reducing felting of wool or another textile made from keratinous fibers is provided, the method comprising contacting the textile with an aqueous composition comprising an enzyme having perhydrolase activity, an ester substrate for the enzyme, and a source of hydrogen peroxide, for an amount of time sufficient time to generate peracids, wherein the peracids modify the textile, thereby reducing the tendency of the textile to felt.

In another particular aspect, a method for reducing felting of wool or another textile made from keratinous fibers is provided, the method comprising: (i) providing in an aqueous composition comprising: an enzyme having perhydrolase activity, an ester substrate for the enzyme, and a source of hydrogen peroxide, (ii) generating peracids *in situ* in the aqueous composition, and (iii) contacting the textile comprising keratinous fibers with the aqueous composition comprising peracids, thereby modifying the textile to reduce the tendency of the textile to felt.

In yet another particular aspect, a method for reducing felting of wool or another textile made from keratinous fibers is provided, the method comprising: (i) providing in an aqueous composition comprising: an enzyme having perhydrolase activity, an ester substrate for the enzyme, and a source of hydrogen peroxide, (ii) generating peracids *in situ* in the aqueous composition, and (iii) contacting keratinous fibers with the aqueous composition comprising peracids, thereby modifying the fibers to reduce the tendency of a textile comprising the fibers to felt.

In a further aspect, in a method for manufacturing a textile comprising keratinous fibers, a method for modifying the fibers to reduce felting of the textile is provided, comprising: (i) providing in an aqueous composition comprising: an enzyme having perhydrolase activity, an ester substrate for the enzyme, and a source of hydrogen peroxide, (ii) generating

peracids *in situ* in the aqueous composition, and (iii) contacting keratinous fibers with the aqueous composition comprising peracids to modifying the fibers, thereby reducing the tendency of a textile comprising the fibers to felt.

In some embodiments of any of the foregoing methods, the keratinous fibers may be obtained from sheep. In some embodiments, the textile is wool.

In some embodiments of any of the foregoing methods, the enzyme having perhydrolase activity is a polypeptide having an amino acid sequence that is at least 70% identical to the amino acid sequence set forth in SEQ ID NO: 1 or SEQ ID NO: 2. In some embodiments, the enzyme having perhydrolase activity may be a polypeptide having an amino acid sequence that is at least 80% identical to the amino acid sequence set forth in SEQ ID NO: 1 or SEQ ID NO: 2. In some embodiments, the enzyme having perhydrolase activity may be a polypeptide having an amino acid sequence that is at least A 90% identical to the amino acid sequence set forth in SEQ ID NO: 1 or SEQ ID NO: 2.

In some embodiments of any of the foregoing methods, the enzyme having perhydrolase activity is derived from *Mycobacterium smegmatis* perhydrolase. In some embodiments, the enzyme having perhydrolase activity is a variant of *Mycobacterium smegmatis* perhydrolase having the substitution S54V.

In some embodiments of any of the foregoing methods, the ester substrate is propylene glycol diacetate (PGDA), triacetin, ethyl acetate, tributyrin, and/or ethylene glycol diacetate (EGDA). In some embodiments, the source of hydrogen peroxide, is hydrogen peroxide, perborate, or percarbonate.

Some embodiments of any of the foregoing methods further comprise the step of treating the keratinous fiber or textiles comprising the keratinous fiber with sodium sulfite to further reduce felting of the textiles. Some embodiments of these methods further comprise the step of treating the keratinous fiber or textiles comprising the keratinous fiber with transglutaminase to improve textile strength.

These and other aspects and embodiments of present methods will be apparent from the description, including the accompanying Figures.

## BRIEF DESCRIPTION OF THE DRAWINGS

Figure 1 is a scanning electron micrograph showing the morphology of wool fibers treated with buffer (magnification ≈ 2,000X).

Figure 2 is a scanning electron micrograph showing the morphology of wool fibers treated with buffer + PGDA + $H_2O_2$ (magnification ≈ 2,000X).

Figure 3 is a scanning electron micrograph showing the morphology of wool fibers treated with buffer + PGDA + $H_2O_2$ + arylesterase (magnification ≈ 2,000X).

Figure 4 shows the FTIR/ATR spectra of wool fibers treated with (1) buffer, (2) buffer + PGDA + $H_2O_2$, and (3) buffer + PGDA + $H_2O_2$ + arylesterase.

Figure 5 shows the results of staining differently-treated wool fibers with Shirlastain A. The fibers on the left are not dyed (Control). The fibers on the right are dyed with Shirlastain A (Dyed).

Figure 6 shows the amount of shrinkage of wool jersey swatches treated with buffer (left), buffer + PGDA + $H_2O_2$ (center), and buffer + PGDA + $H_2O_2$ + arylesterase (right).

Figure 7 illustrates the location of reference marks (*i.e.*, cotton/polyester threads; shown as crosses) placed on wool knit samples prior to treatment.

Figure 8 shows the sizes of wool interlock swatches treated with PGDA + $H_2O_2$ (left) and PGDA + $H_2O_2$ + arylesterase (right) and the washed as described.

Figures 9 and 10 are scanning electron micrographs showing the morphology of wool fibers from swatches treated with buffer + PGDA + $H_2O_2$ (Figure 9) and buffer + PGDA + $H_2O_2$ + arylesterase (Figure 10), (magnification = 2,000X).

Figure 11 shows the FTIR/ATR spectra of control wool fibers and those treated with enzymatically-generated peracids.

Figure 12 shows the FTIR/ATR spectra of wool jersey knit treated with (1) no enzyme (2) aryl esterase, (3) sodium sulfite, (4) no enzyme followed by sodium sulfite, and (4) aryl esterase followed by sodium sulfite.

## DETAILED DESCRIPTION

### *Definitions*

Prior to describing the present methods in detail, the following terms are defined for clarity. Terms not defined should be accorded their ordinary meanings as using in the relevant art.

As used herein, "keratinous fibers" are fibers comprising keratin, a family of fibrous structural proteins found naturally in animal cells. Keratinous fibers are primarily found in wool (see, below), hair, fur, nails, and other animal

tissues. As used herein, keratinous fibers include both naturally occurring and synthetic fibers.

As used herein, "wool" refers to textiles made from keratinous fibers of animals in the Caprinae family, principally sheep. However, wool also encompasses cashmere and mohair from goats, vicuña, alpaca, and camel from animals in the camel family, and angora from rabbits. The keratinous fibers used to make wool are similar to but distinct from hair or fur.

As used herein, "felting" refers to the shrinkage of textiles comprising keratinous fibers in one or more dimensions. Felting is readily caused, e.g., by manipulating such textiles in a wet state, wherein scales present on the surface of the keratinous fibers interact, drawing the fibers together, and shrinking the textiles.

As used herein, "shrinkage" refers to a decrease in the surface area of a fabric as measured in one or more dimensions. Shrinking may be associated with an increase in density and changes to texture.

As used herein, the term "textile" refers to fibers, yarns, fabrics, garments, and non-wovens. Textiles may natural, synthetic (e.g., manufactured), or blends, thereof. The term "textile" refers to both unprocessed and processed fibers, yarns, woven or knit fabrics, non-wovens, and garments.

As used herein, the term "fabric" refers to a manufactured assembly of fibers and/or yarns that has substantial surface area in relation to its thickness and sufficient cohesion to give the assembly useful mechanical strength.

As used herein, the term "dye" refers to a colored substance *(i.e.,* chromophore) that has an affinity for a substrate, such as a textile, to which it is applied.

As used herein, the term "dyeing," refers to applying a chromophore to a substrate, such as a textile, especially by soaking in a dyeing solution.

As used herein, an "aqueous medium" is a solution or mixed solution/suspension in which the solvent is primarily water. An aqueous medium is substantially free of inorganic solvents.

As used herein, a "perhydrolase enzyme" or a "perhydrolase" is an enzyme capable of catalyzing a perhydrolysis reaction that results in the production of peracids. Preferably, the perhydrolase enzyme exhibits a high perhydrolysis to hydrolysis ratio. Perhydrolases may possess acyl transferase and/or aryl esterase activity, and may be named, accordingly.

As used herein, the term "perhydrolysis" refers to a reaction in which an ester substrate and hydrogen peroxide are used to may peracids.

As used herein, an "effective amount of perhydrolase enzyme" refers to the quantity of perhydrolase enzyme necessary to modify keratinous fibers to reduce felting of a fabric made thereform.

As used herein, the term "peracid" refers to a molecule derived from a carboxylic acid ester that has been reacted with hydrogen peroxide to form a highly reactive product having the general formula RC(=O)OOH. Such peracid products are able to transfer one of their oxygen atoms to another molecule, such as keratinous fiber.

As used herein, the term "ester substrate" refers to a perhydrolase substrate that contains at least one ester linkage. Esters comprising aliphatic and/or aromatic carboxylic acids and alcohols may be utilized as substrates with perhydrolase enzymes.

As used herein, the term "acyl" refers to an organic group with the general formula RCO-, which may be derived from an organic acid by removal of the -OH group. Typically, acyl group names end with the suffix "-oyl," e.g., methanoyl chloride, $CH_3CO$-Cl, is the acyl chloride formed from methanoic acid, $CH_3CO$-OH).

As used herein, the term "acylation" refers to a chemical transformation in which one of the substituents of a molecule is substituted by an acyl group, or the process of introduction of an acyl group into a molecule.

As used herein, the term "hydrogen peroxide source" refers to a molecule capable of generating hydrogen peroxide, *e.g., in situ.* Hydrogen peroxide sources include hydrogen peroxide, itself, as well as molecules that spontaneously or enzymatically produce hydrogen peroxide as a reaction product.

As used herein, the phrase "perhydrolysis to hydrolysis ratio" refers to the ratio of enzymatically produced peracid to enzymatically produced acid (*e.g*., in moles) that is produced by a perhydrolase enzyme from an ester substrate under defined conditions and within a defined time. The assays provided in WO 05/056782 may be used to determine the amounts of peracid and acid produced by the enzyme.

As used herein, the term "hydrogen peroxide generating oxidase" refers to an enzyme that catalyzes an oxidation/reduction reaction involving molecular oxygen ($O_2$) as the electron acceptor. In such a reaction, oxygen is reduced to water ($H_2O$) or hydrogen peroxide ($H_2O_2$). An oxidase suitable for use herein is an oxidase that generates hydrogen peroxide (as opposed to water) on its substrate. An example of a hydrogen peroxide generating oxidase and its substrate suitable for use herein is glucose oxidase and glucose. Other oxidase enzymes that may be used for generation of hydrogen peroxide include alcohol oxidase, ethylene glycol oxidase, glycerol oxidase, amino acid oxidase, etc. The hydrogen peroxide generating oxidase may be a carbohydrate oxidase.

As used herein, the terms "polypeptide" and "protein" are used interchangeably to refer to polymers of any length comprising amino acid residues linked by peptide bonds. The conventional one-letter or three-letter code for amino acid residues is used herein. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation,

or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, functionally and/or structurally similar proteins are considered to be "related proteins." Such proteins may be derived from organisms of different genera and/or species, or even different classes of organisms (e.g., bacteria and fungus). Related proteins also encompass homologs determined by primary sequence analysis, determined by tertiary structure analysis, or determined by immunological cross-reactivity.

As used herein, the term "derivative polypeptide/protein" refers to a protein which is derived from a protein by addition of one or more amino acids to either or both the N- and C-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative may be achieved by modifying a DNA sequence which encodes for the native protein, transformation of that DNA sequence into a suitable host, and expression of the modified DNA sequence to form the derivative protein.

Related (and derivative) proteins include "variant proteins." Variant proteins differ from a reference/parent protein, *e.g.*, a wild-type protein, by substitutions, deletions, and/or insertions at small number of amino acid residues. The number of differing amino acid residues may be one or more, for example, 1, 2, 3, 4, 5, 6, 7, 8, 9, 10, 15, 20, 30, 40, 50, or more amino acid residues. Variant proteins share at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or even at least about 99%, or more, amino acid sequence identity with a reference protein. A variant protein may also differ from a reference protein in selected motifs, domains, epitopes, conserved regions, and the like.

As used herein, the term "analogous sequence" refers to a sequence within a protein that provides similar function, tertiary structure, and/or conserved residues as the protein of interest (*i.e.,* typically the original protein of interest). For example, in epitope regions that contain an alpha-helix or a beta-sheet structure, the replacement amino acids in the analogous sequence preferably maintain the same specific structure. The term also refers to nucleotide sequences, as well as amino acid sequences. In some embodiments, analogous sequences are developed such that the replacement amino acids result in a variant enzyme showing a similar or improved function. In some embodiments, the tertiary structure and/or conserved residues of the amino acids in the protein of interest are located at or near the segment or fragment of interest. Thus, where the segment or fragment of interest contains, for example, an alpha-helix or a beta-sheet structure, the replacement amino acids preferably maintain that specific structure.

As used herein, the term "homologous protein" refers to a protein that has similar activity and/or structure to a reference protein. It is not intended that homologs necessarily be evolutionarily related. Thus, it is intended that the term encompass the same, similar, or corresponding enzyme(s) *(i.e.,* in terms of structure and function) obtained from different organisms. In some embodiments, it is desirable to identify a homolog that has a quaternary, tertiary and/or primary structure similar to the reference protein. In some embodiments, homologous proteins induce similar immunological response(s) as a reference protein. In some embodiments, homologous proteins are engineered to produce enzymes with desired activity(ies).

The degree of homology between sequences may be determined using any suitable method known in the art (*see, e.g.,* Smith and Waterman (1981) Adv. Appl. Math. 2:482; Needleman and Wunsch (1970) J. Mol. Biol., 48:443; Pearson and Lipman (1988) Proc. Natl. Acad. Sci. USA 85:2444; programs such as GAP, BESTFIT, FASTA, and TFASTA in the Wisconsin Genetics Software Package (Genetics Computer Group, Madison, WI); and Devereux et al. (1984) Nucleic Acids Res. 12:387-395).

For example, PILEUP is a useful program to determine sequence homology levels. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle, (Feng and Doolittle (1987) J. Mol. Evol. 35:351-360). The method is similar to that described by Higgins and Sharp (Higgins and Sharp (1989) CABIOS 5:151-153). Useful PILEUP parameters including a default gap weight of 3.00, a default gap length weight of 0.10, and weighted end gaps. Another example of a useful algorithm is the BLAST algorithm, described by Altschul *et al.* (Altschul et al. (1990) J. Mol. Biol. 215:403-410; and Karlin et al. (1993) Proc. Natl. Acad. Sci. USA 90:5873-5787). One particularly useful BLAST program is the WU-BLAST-2 program (See, Altschul et al. (1996) Meth. Enzymol. 266:460-480). Parameters "W," "T," and "X" determine the sensitivity and speed of the alignment. The BLAST program uses as defaults a word-length (W) of 11, the BLOSUM62 scoring matrix (See, Henikoff and Henikoff (1989) Proc. Natl. Acad. Sci. USA 89:10915) alignments (B) of 50, expectation (E) of 10, M'5, N'-4, and a comparison of both strands.

As used herein, the phrases "substantially similar" and "substantially identical," in the context of at least two nucleic acids or polypeptides, typically means that a polynucleotide or polypeptide comprises a sequence that has at least about 70% identity, at least about 75% identity, at least about 80% identity, at least about 85% identity, at least

about 90% identity, at least about 91 % identity, at least about 92% identity, at least about 93% identity, at least about 94% identity, at least about 95% identity, at least about 96% identity, at least about 97% identity, at least about 98% identity, or even at least about 99% identity, or more, compared to the reference (*i.e.,* wild-type) sequence. Sequence identity may be determined using known programs such as BLAST, ALIGN, and CLUSTAL using standard parameters. (*See e.g.,* Altschul, et al. (1990) J. Mol. Biol. 215:403-410; Henikoff et al. (1989) Proc. Natl. Acad. Sci. USA 89:10915; Karin et al. (1993) Proc. Natl. Acad. Sci USA 90:5873; and Higgins et al. (1988) Gene 73:237-244). Software for performing BLAST analyses is publicly available through the National Center for Biotechnology Information. Also, databases may be searched using FASTA (Pearson et al. (1988) Proc. Natl. Acad. Sci. USA 85:2444-2448). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (e.g., within a range of medium to high stringency).

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project. The genes encoding the naturally-occurring protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

As used herein, "packaging" refers to a container capable of providing a perhydrolase enzyme, substrate for the perhydrolase enzyme, and/or hydrogen peroxide source in an easy to handle and transport form. Exemplary packaging includes boxes, tubs, cans, barrels, drums, bags, or even tanker trucks.

As used herein, the term "contacting" means incubating in the presence of, typically in aqueous solution.

As used herein, the term "modifying a keratinous fiber" refers to any chemical alteration of a keratinous fiber that results in decreased felting of a fabric comprising the modified fiber. Chemical modifications include but are not limited to oxidation, formation of cysteic acid, and the formation of Bunte salts.

As used herein, the singular articles "a," "an," and "the" encompass the plural referents unless the context clearly dictates otherwise.

The following abbreviations/acronyms have the following meanings unless otherwise specified:

| | |
|---|---|
| EC | enzyme commission |
| kDa | kiloDalton |
| MW | molecular weight |
| w/v | weight/volume |
| w/w | weight/weight |
| v/v | volume/volume |
| wt% | weight percent |
| °C | degrees Centigrade |
| $H_2O$ | water |
| $H_2O_2$ | hydrogen peroxide |
| $dH_2O$ or DI | deionized water |
| $dIH_2O$ | deionized water, Milli-Q filtration |
| g or gm | gram |
| μg | microgram |
| mg | milligram |
| kg | kilogram |
| lb | pound |
| μL and μl | microliter |
| mL and ml | milliliter |
| mm | millimeter |
| μm | micrometer |
| M | molar |

(continued)

| | |
|---|---|
| mM | millimolar |
| μM | micromolar |
| U | unit |
| ppm | parts per million |
| sec | second |
| min | minute |
| " | inches |
| hr | hour |
| EtOH | ethanol |
| eq. | equivalent |
| N | normal |
| CI | Colour (Color) Index |
| CAS | Chemical Abstracts Society |

***Overview***

The present methods relate to the chemical modification of keratinous fibers to reduce the amount of felting of a fabric comprising such fibers. A feature of the methods is the treatment of keratinous fibers with enzymatically-generated peracids in an aqueous medium, thereby obviating many problems associated with conventional methods.

In one aspect, the methods involve contacting keratinous fibers, or a fabric made from keratinous fibers, with an aqueous composition comprising an enzyme having perhydrolase activity, an ester substrate for the enzyme, and a source of hydrogen peroxide, for an amount of time sufficient time to generate peracids. As the *in situ*-generated peracids are produced, they modify the keratinous fibers in an aqueous environment, thereby reducing the tendency of a fabric made from the keratinous fibers to felt.

In another aspect, the claimed methods comprise use of an aqueous composition comprising an enzyme having perhydrolase activity, an ester substrate for the enzyme, and a source of hydrogen peroxide, for generating peracids *in situ* in an aqueous composition. Keratinous fibers or a fabric made from keratinous fibers are then contacted with the *in situ*-generated peracids under aqueous conditions, wherein the peracids modify the fibers and reduce the tendency of a fabric made from the fabrics to felt.

Various features and preferred embodiments of the methods are to be described.

***Perhydrolase Enzyme***

A feature of the present methods for reducing the felting of fabrics made from keratinous fibers is the use of one or more perhydrolase enzymes. Generally, perhydrolase enzymes are capable of generating peracids from suitable ester substrates in the presence of hydrogen peroxide.

In some embodiments, the perhydrolase enzyme is naturally-occurring enzyme, or a perhydrolase enzyme comprising, consisting of, or consisting essentially of an amino acid sequence that is at least about 70%, 75%, 80%, 85%, 90%, 91%, 92%, 93%, 94%, 95%, 96%, 97%, 98%, 99%, or even 99.5% identical to the amino acid sequence of a naturally-occurring perhydrolase enzyme. The perhydrolase enzyme may be from a microbial source, such as a bacterium or fungus.

In some embodiments, the perhydrolase enzyme is a naturally occurring *Mycobacterium smegmatis* perhydrolase enzyme or a variant thereof. This enzyme, its enzymatic properties, its structure, and numerous variants and homologs, thereof, are described in detail in International Patent Application Publications WO 05/056782A and WO 08/063400A and U.S. Patent Application Publications US2008145353 and US2007167344.

In some embodiments, a perhydrolase enzyme comprises, consists of, or consists essentially of the amino acid sequence set forth in SEQ ID NO: 1 or a variant or homologue thereof. In some embodiments, the perhydrolase enzyme comprises, consists of, or consists essentially of an amino acid sequence that is at least about 70%, 75%, 80%, 85%, 90%, 91%, 92%, 93%, 94%, 95%, 96%, 97%, 98%, 99%, or even 99.5% identical to the amino acid sequence set forth in SEQ ID NO: 1.

The amino acid sequence of M. *smegmatis* perhydrolase is shown below (SEQ ID NO: 1):

MAKRILCFGDSLTWGWVPVEDGAPTERFAPDVRWTGVLAQQLGADFEVIEEGLSAR
TTNIDDPTDPRLNGASYLPSCLATHLPLDLVIIMLGTNDTKAYFRRTPLDIALGMSVLVT
QVLTSAGGVGTTYPAPKVLVVSPPPLAPMPHPWFQLIFEGGEQKTTELARVYSALAS
FMKVPFFDAGSVISTDGVDGIHFTEANNRDLGVALAEQVRSLL

In some embodiments, the perhydrolase enzyme may comprise one or more A substitutions at one or more amino acid positions equivalent to position(s) in the M. *smegmatis* perhydrolase amino acid sequence set forth in SEQ ID NO: 1. In some embodiments, the perhydrolase enzyme may comprise any one or any combination of substitutions of amino acids selected from M1, K3, R4, 15, L6, C7, D10, S11, L12, T13, W14, W16, G15, V 17, P 18, V 19, D21, G22, A23, P24, T25, E26, R27, F28, A29, P30, D31, V32, R33, W34, T35, G36, L38, Q40, Q41, D45, L42, G43, A44, F46, E47, V48, I49, E50, E51, G52, L53, S54, A55, R56, T57, T58, N59, I60, D61, D62, P63, T64, D65, P66, R67, L68, N69, G70, A71, S72, Y73, S76, C77, L78, A79, T80, L82, P83, L84, D85, L86, V87, N94, D95, T96, K97, Y99F100, R101, R102, P104, L105, D106, I107, A108, L109, G110, M111, S112, V113, L114, V115, T116, Q117, V118, L119, T120, S121, A122, G124, V125, G126, T127, T128, Y129, P146, P148, W149, F150, 1153, F154, I194, and F196.

In some embodiments, the perhydrolase enzyme may comprise one or more of the following substitutions at one or more amino acid positions equivalent to position(s) in the *M. smegmatis* perhydrolase amino acid sequence set forth in SEQ ID NO: 1: L12C, Q, or G; T25S, G, or P; L53H, Q, G, or S; S54V, L A, P, T, or R; A55G or T; R67T, Q, N, G, E, L, or F; K97R; V125S, G, R, A, or P; F154Y; F196G.

In some embodiments, the perhydrolase enzyme may comprise a combination of amino acid substitutions at amino acid positions equivalent to amino acid positions in the *M. smegmatis* perhydrolase amino acid sequence set forth in SEQ ID NO: 1: L12I S54V; L12M S54T; L12T S54V; L12Q T25S S54V; L53H S54V; S54P V125R; S54V V125G; S54V F196G; S54V K97R V125G; or A55G R67T K97R V125G.

In particular embodiments, the perhydrolase enzyme is the S54V variant of the *M. smegmatis* perhydrolase, which is shown, below (SEQ ID NO: 2; S54V substitution underlined):

MAKRILCFGDSLTWGWVPVEDGAPTERFAPDVRWTGVLAQQLGADFEVIEEGLVAR
TTNIDDPTDPRLNGASYLPSCLATHLPLDLVIIMLGTNDTKAYFRRTPLDIALGMSVLVT
QVLTSAGGVGTTYPAPKVLVVSPPPLAPMPHPWFQLIFEGGEQKTTELARVYSALAS
FMKVPFFDAGSVISTDGVDGIHFTEANNRDLGVALAEQVRSLL

In some embodiments, the perhydrolase enzyme includes the S54V substitution but is otherwise at least about 70%, 75%, 80%, 85%, 90%, 91%, 92%, 93%, 94%, 95%, 96%, 97%, 98%, 99%, or even 99.5% identical to the amino acid sequence set forth in SEQ ID NOs: 1 or 2.

In some embodiments, the perhydrolase enzyme may have a perhydrolysis:hydrolysis ratio of at least 1. In some embodiments, a perhydrolase enzyme may have a perhydrolysis:hydrolysis ratio greater than 1.

The amount of perhydrolase enzyme is generally not critical, and is selected to generate a sufficient amount of peracids to modify the keratinous fibers in the manner described.

***Ester Substrate***

Another feature of the present methods is the use of an ester substrate for the perhydrolase enzyme for production of a peracid in the presence of hydrogen peroxide. Suitable substrates may be monovalent (*i.e.,* comprising a single carboxylic acid ester moiety) or plurivalent (*i.e.,* comprising more than one carboxylic acid ester moiety). The amount of substrate required for peracid generation may be adjusted depending on the number carboxylic acid ester moieties in the substrate molecule.

In some embodiments, the ester substrate is an ester of an aliphatic and/or aromatic carboxylic acid or alcohol. The ester substrate may be a mono-, di-, or multivalent ester, or a mixture thereof. For example, the ester substrate may be a carboxylic acid and a single alcohol (monovalent, e.g., ethyl acetate, propyl acetate), two carboxylic acids and a diol [e.g., propylene glycol diacetate (PGDA), ethylene glycol diacetate (EGDA), or a mixture, for example, 2-acetyloxy 1-propionate, where propylene glycol has an acetate ester on alcohol group 2 and a propyl ester on alcohol group 1], or three carboxylic acids and a triol (e.g., glycerol triacetate or a mixture of acetate/propionate, etc., attached to glycerol

or another multivalent alcohol). In some embodiments, the ester substrate may be an ester of a nitroalcohol (e.g., 2-nitro-1-propanol). In some embodiments, the ester substrate is a polymeric ester, for example, a partially acylated (acetylated, propionylated, etc.) poly carboxy alcohol, acetylated starch, etc. In some embodiments, the ester substrate may be an ester of one or more of the following: formic acid, acetic acid, propionic acid, butyric acid, valeric acid, caproic acid, caprylic acid, nonanoic acid, decanoic acid, dodecanoic acid, myristic acid, palmitic acid, stearic acid, and oleic acid. In some embodiments, triacetin, tributyrin, and other esters serve as acyl donors for peracid formation. In some embodiments, the ester substrate is propylene glycol diacetate, ethylene glycol diacetate, or ethyl acetate. In one embodiment, the ester substrate is propylene glycol diacetate.

The amount of ester substrate is generally not critical, and is selected to generate a sufficient amount of peracids to modify the keratinous fibers in the manner described.

***Hydrogen Peroxide Source***

Another feature of the present methods is the use of a hydrogen peroxide source. Generally, hydrogen peroxide can be provided directly or generated continuously by chemical, electro-chemical, and/or enzymatic means.

In some embodiments, the hydrogen peroxide source may be hydrogen peroxide, itself. In some embodiments, the hydrogen peroxide source may be a compound that generates hydrogen peroxide upon addition to water. The compound may be a solid compound. Such compounds include adducts of hydrogen peroxide with various inorganic or organic compounds, of which the most widely employed is sodium carbonate per hydrate, also referred to as sodium percarbonate.

In some embodiments, the hydrogen peroxide source may be an inorganic perhydrate salt. Examples of inorganic perhydrate salts are perborate, percarbonate, perphosphate, persulfate and persilicate salts. Inorganic perhydrate salts are normally alkali metal salts.

Additional hydrogen peroxide sources include adducts of hydrogen peroxide with zeolites, or urea hydrogen peroxide.

The hydrogen peroxide source may be in a crystalline form and/or substantially pure solid form without additional protection. For certain perhydrate salts, preferred forms are granular compositions involving a coating, which provides better storage stability for the perhydrate salt in the granular product. Suitable coatings comprise inorganic salts such as alkali metal silicate, carbonate or borate salts or mixtures thereof, or organic materials such as waxes, oils, or fatty soaps.

In some embodiments, the hydrogen peroxide source may be an enzymatic hydrogen peroxide generation system. In one embodiment, the enzymatic hydrogen peroxide generation system may comprise an oxidase and its substrate. Suitable oxidase enzymes include, but are not limited to: glucose oxidase, sorbitol oxidase, hexose oxidase, choline oxidase, alcohol oxidase, glycerol oxidase, cholesterol oxidase, pyranose oxidase, carboxyalcohol oxidase, L-amino acid oxidase, glycine oxidase, pyruvate oxidase, glutamate oxidase, sarcosine oxidase, lysine oxidase, lactate oxidase, vanillyl oxidase, glycolate oxidase, galactose oxidase, uricase, oxalate oxidase, and xanthine oxidase.

The following equation provides an example of a coupled system for enzymatic production of hydrogen peroxide.

$$\text{Glucose} + H_2O \xrightarrow{\text{Glucose oxidase}} \text{gluconic acid} + H_2O_2$$

$$+$$

$$H_2O_2 + \text{ester substrate} \xrightarrow{\text{Perhydrolase}} \text{alcohol} + \text{peracid}$$

It is not intended that the generation of $H_2O_2$ be limited to any specific enzyme, as any enzyme that generates $H_2O_2$ with a suitable substrate may be used. For example, lactate oxidases from *Lactobacillus* species known to create $H_2O_2$ from lactic acid and oxygen may be used. One advantage of such a reaction is the enzymatic generation of acid (e.g., gluconic acid in the above example), which reduces the pH of a basic aqueous solution to within the pH range in which peracid is most effective in bleaching (i.e., at or below the pKa). Such a reduction in pH is also brought about directly by the production of peracid. Other enzymes (e.g., alcohol oxidase, ethylene glycol oxidase, glycerol oxidase, amino acid oxidase, etc.) that are capable of generating hydrogen peroxide may also be used with ester substrates in combination with a perhydrolase enzyme to generate peracids.

Where hydrogen peroxide is generated electrochemically, it may be produced, for example, using a fuel cell supplied with oxygen and hydrogen gas.

The amount of hydrogen peroxide is generally not critical, and is selected to generate a sufficient amount of peracids to modify the keratinous fibers in the manner described. Following treatment of keratinous fibers, it may in some circumstances be desirable to use a catalase enzyme to destroy residual hydrogen peroxide.

### *Fibers and Fabrics*

Keratinous fibers suitable for treatment as described include both naturally occurring fibers and synthetic fibers that comprise polypeptides classified as keratins, a family of fibrous structural proteins found mainly in animal cells. Keratins are rich in cysteine, as disulfide bridges between cysteine residues are important for the overall structural integrity of keratinous fibers. Natural products comprising keratinous fibers include, but are not limited to, wool, hair, fur, nails, horns, hooves, feathers, and skin. In some cases, keratin comprises the majority of the dry weight of these materials. Although keratinous fibers are generally obtained from natural sources, nothing in the present description should be taken to exclude the use of keratinous fibers made from extracted keratin protein or even recombinant keratin protein.

The term "wool" is sometimes used to refer only to keratinous fibers derived from specialized skin cells (called follicles) of sheep. However, the term "wool" is also used to refer more generally to keratinous fibers derived from animals in the Caprinae family, including sheep, goats, camels, and rabbits. Wool from goats is also known as cashmere and mohair; wool from camels is also known vicuña and alpaca, and wool from rabbits is also known as angora. The present methods are useful for treating wool, generally, although they are exemplified using sheep wool.

Keratinous fibers may be treated prior to incorporation into a textile, after incorporation into a yarn, fabric, textile, or garment, or both. Such treatment may be performed in aqueous media in a batch or a continuous manner. Fabrics comprising keratinous fibers may be blended with other fibers, including other natural or synthetic fibers. Exemplary fibers include but are not limited to cotton, linen, polyester, polyamide, hemp, and the like.

### *Dyes*

A feature of the present methods is the ability to increase dye uptake by keratinous fibers, or a fabric made therefrom. Examples of suitable dyes include, but are not limited to, azo, monoazo, disazo, nitro, xanthene, quinoline, anthroquinone, triarylmethane, paraazoanyline, azineoxazine, stilbene, aniline, and phthalocyanine dyes, or mixtures thereof. The dye may be an azo dye (e.g., Reactive Black 5 (2,7-naphthalenedisulfonic acid, 4-amino-5- hydroxy-3,6-bis((4-((2-(sulfooxy)ethyl)sulfonyl)phenyl)azo)-tetrasodium salt), Reactive Violet 5, methyl yellow, Congo red, etc.), an anthraquinone dye (e.g., remazol blue), indigo (indigo carmine), a triarylmethane/paraazoanyline dye (e.g., crystal violet, malachite green), or a sulphur-based dye. The dye may be a reactive, direct, disperse, or pigment dye. The dye may be a component of an ink.

### *Aqueous Medium*

A feature of the present methods is that keratinous fibers, or fabrics made from keratinous fibers, are treated with enzymatically-generated peracids in aqueous media. Aqueous medium is a solution or mixed solution/suspension in which the primary solvent is water. This feature readily distinguishes the present methods from conventional peracid treatment of keratinous fibers, which was performed in substantially inorganic solvents (see, e.g., U.S. Patent No. 3,634,020). While small amount of such solvents are generally tolerated in the present methods, they are not required. Accordingly, the aqueous medium may be entirely free of organic solvents, or may contain less than 5%, less than 4%, less than 3%, less than 2%, less than 1%, less than 0.7%, less than 0.5%, less than 0.2%, or even less than 0.1% organic solvents.

Aqueous media may include any number of buffers, surfactants, salts, lubricants, binders, builders, polymers, chelating agents, complexing agents, antifouling agents, dyes, and the like. Aqueous media may further include additional enzymes, substrates, cofactors, activators, etc. for such enzymes. However, in some embodiments, the aqueous medium expressly may not include significant amounts proteases and/or peroxidases. Suitable aqueous media is that used for textile processing, which typically includes buffers, salts, and optionally surfactants.

### *Sodium Sulfite Treatment*

In some embodiments of the present methods, the keratinous fibers, or textiles comprising such fibers, are additionally treated with sodium sulfite. Treatment with sodium sulfite (or other sulfite salts) is known to cause the formation of Bunte salts with oxidized cysteine residues. Conventional methods of Bunte salt formation rely on the treatment of keratinous fibers with a strong oxidizing agent, such as peracetic acid, permanganeate, persulfate, hydrogen peroxide, and the like, followed by treatment with sodium sulfite.

In some embodiments of the present methods, keratinous fibers or fabrics comprising keratinous fibers are treated first with enzymatically-generated peracids in aqueous medium, followed by treatment with sodium sulfite. As exemplified, herein treatment with enzymatically peracid in aqueous medium followed by sodium sulfite treatment resulted in even less felting than treatment with enzymatically peracid alone.

### *Transglutaminase Treatment*

In some embodiments of the present methods, a transglutaminase is used to crosslink enzymatically-generated peracid-treated or enzymatically-generated peracid and sodium sulfite-treated keratinous fibers, or textiles comprising such fibers. Such treatment is described in detail in, e.g., Cortez et al. (2004) Enzyme and Microbial Technology 34:64-72. Transglutaminase treatment can increase the strength of wool or other textiles made from keratinous fibers, or restore strength lost during treatment with enzymatically-generated peracids.

These and other aspects and embodiments of the present method will be apparent to the skilled person in view of the present description. The following examples are intended to further illustrate, but not limit, the methods.

## EXAMPLES

### Example 1: Change in the Morphology of Enzymatically-treated Wool Fibers

50 mg of 100% wool fibers *(i.e.,* "tops") were incubated in a 2 ml reaction volume at 65°C in a glass tube with slow agitation for one hour. The incubation conditions were as follows:

1) Buffer (100 mM sodium phosphate buffer, pH 7),
2) Buffer + 5 μl/ml PGDA + 5 μl/ml $H_2O_2$ (50% w/w), or
3) Buffer + 5 μl/ml PGDA + 5 μl/ml $H_2O_2$ (50% w/w) + 0.8 ppm perhydrolase enzyme.

The particular perhydrolase enzyme used was the S54V variant of M. *smegmatis* perhydrolase (also referred to as "arylesterase"). The wool fibers were rinsed with DI water 3 times following treatment, and then air-dried. The morphology of the treated wool fibers was evaluated using the Phenom scanning electron microscopy by FEI.

Treatment of the wool fibers with buffer + PGDA+ $H_2O_2$ (Figure 2) produced no significant changes on wool scale morphologies compared to treatment only with buffer (Figure 1). However, the addition of perhydrolase enzyme resulted in significant removal of wool scales (Figure 3).

Chemical changes in the wool fibers resulting from the different treatments were monitored by Fourier Transform Infrared Spectroscopy in the Attenuated Total Reflection Mode (FTIR/ATR). Solid wool fibers were directly mounted on the MIRacle ATR accessory attached to Tensometer 27 (Brucker Optics) to obtain spectrums after the different treatments. As shown in Figure 4, large increases in the spectrum at approximately at 1040 $cm^{-1}$ and 1170 $cm^{-1}$ indicate an increase in the levels of cysteic acid (an oxidation product of cysteine) following treatment with the perhydrolase enzyme (Figure 4).

These results suggest that treatment with the perhydrolase enzyme reduces the surface scaling of wool fibers, possibly as a consequence of oxidizing cysteine residue present in wool proteins.

### Example 2: Change in the Dyeing Affinity of Enzymatically-treated Wool Fibers

50 mg of treated wool fibers (see Example 1) were stained with Shirlastain A (a combination of picric acid, G150 Chlorazol Blue, and 3BS Crocein Scarlet) at room temperature for 5 minutes. A significant increase in dyeing affinity using Shirlastain A was observed in the wool fibers treated with the perhydrolase enzyme (Figure 5). While wool fibers treated with buffer or buffer + PGDA + $H_2O_2$ dyed a medium orange color, wool fibers treated with buffer or buffer + PGDA + $H_2O_2$ + perhydrolase enzyme dyed an intense red color. These results demonstrate that treatment of the wool fibers with the perhydrolase enzyme increases dye uptake.

### Example 3: Reduced Shrinkage of Treated Wool Knit Fabric in a Launder-O-meter

3 each 100% wool jersey swatches (measuring 5" x 5"; Testfabrics style 532) were incubated in a 300 ml reaction volume (liquor ratio = 32:1) in a Launder-O-meter for one hour at 65°C under one the following conditions (n=3):

1) Buffer (100 mM sodium phosphate buffer, pH 7),
2) Buffer + 5 μl/ml PGDA + 5 μl/ml $H_2O_2$ (50% w/w), or
3) Buffer + 5 μl/ml PGDA + 5 μl/ml $H_2O_2$ (50% w/w) + 0.8 ppm perhydrolase enzyme.

Representative results are shown in Figure 6. The swatches treated with perhydrolase enzyme showed significantly less shrinkage compared to the swatches treated with buffer only or buffer + PGDA + $H_2O_2$. These results demonstrate that treatment of wool fibers with perhydrolase enzyme decreases shrinkage under wash conditions.

**Example 4: Reduced Shrinkage of Treated Wool Knit Fabric in a Unimac**

5 each 100% wool interlock swatches (Britannia Mills LTD, style 7061) were treated in a 30 L reaction volume (liquor ratio = about 51:1) in a Unimac (*i.e.,* 50 lb laboratory-scale tumbling washer) for one hour at 60°C with very gentle agitation (7 RPM) under one the following conditions (n=5):

1) Buffer + 5 ml/L PGDA + 5 ml/L $H_2O_2$ (50% w/w)
2) Buffer + 5 ml/L PGDA + 5 ml/L $H_2O_2$ (50% w/w) + 0.8 ppm perhydrolase enzyme

The buffer was 100 mM sodium phosphate buffer (pH 7) with 0.05 ml/L Triton X-100 (10% w/w) added as a wetting agent. Prior to treatment, each knit specimen (30 cm x 40 cm; longer in machine direction) was marked with small knots of cotton/polyester thread as shown in Figure 7. These marks served as reference points to facilitate the measurement of shrinkage following simulated washing.

Following washing, the wool swatches were subjected to three rinse cycles (incoming water), and then air dried flat before performing felting shrinkage tests. Representative swatches are shown in Figure 8. The swatches treated with the perhydrolase enzyme were much larger than the control swatches following washing, demonstrating that treatment with the perhydrolase enzyme reduced wool shrinkage, even in a 50 lb-scale washer.

Scanning electron micrographs of the control and perhydrolase enzyme-treated swatches are shown in Figures 9 and 10, respectively. The fibers of the perhydrolase enzyme-treated swatches exhibited significantly altered morphology (*e.g.,* reduced scaling) compared to the fibers of the control swatches. FTIR/ATR analysis of untreated fibers, fibers from swatches treated with buffer + PGDA + $H_2O_2$, and fibers from swatches treated with buffer + PGDA + $H_2O_2$ + perhydrolase enzyme, again showed modification resulting from perhydrolase enzyme treatment.

Relaxation shrinkage of the control and perhydrolase enzyme-treated wool interlock swatches was measured according to IWS TM 31,1 X7A and felting shrinkage was measured according to modified IWS TM 31, 5X5A methods. The Unimac (Model UY230, 50-lb laboratory scale tumbling dye machine with microprocessor) was used for the treatments.

Treated specimens were flat dried and the percent shrinkage was calculated based on the method listed in IWS TM31. These shrinkage calculations are summarized, below:

<u>Shrinkage Calculations:</u>

$$\text{Relaxation Shrinkage (\%)} = \{(\text{O.M.}-\text{R.M.})/\text{O.M.}\} \times 100$$

$$\text{Felting Shrinkage (\%)} = \{(\text{R.M.}-\text{F.M.})/\text{R.M.}\} \times 100$$

Where:

O.M. = Original measurements
R.M. = Relaxed measurements
F.M. = Felted measurements

As shown in the table in Figure 13, the perhydrolase enzyme-treated wool knit fabrics showed dramatically lower felting and area shrinkage compared to the control treatment without perhydrolase enzyme treatment. The negative number indicates that the fabric actually increased in length following treatment.

**Example 5: Enzymatic Treatment Combined with Sodium Sulfite Treatment on Wool Knit Fabric**

4 each 100% wool jersey swatches (29" x 24"; Testfabrics, style 532) were treated in a 30 L reaction volume (liquor ratio = about 75:1) in a Unimac 50-lb tumbling washer for 1 hour at 65°C with very gentle agitation (7 RPM) under the following conditions (n=4):

1) Buffer + 5 ml/L PGDA + 5 ml/L $H_2O_2$ (50% w/w)
2) Buffer + 5 ml/L PGDA + 5 ml/L $H_2O_2$ (50% w/w) + 0.8 ppm perhydrolase enzyme.

The buffer was 100 mM sodium phosphate buffer (pH 7) with 0.05 ml/L Triton X-100 (10% w/w) added as a wetting agent. 30 cm x 40 cm rectangles (longer in the machine direction) were marked with small knots of cotton/polyester thread on each knit swatch, as illustrated in Figure 7.

Following treatment, three rinses were performed and the samples were flat dried. The control and enzymatically treated swatches, plus two additional untreated jersey swatches, were incubated with 4g/L of sodium sulfite (along with two untreated jersey fabrics as controls) at 40°C and pH 7 (50 mM sodium phosphate) for one hour in the Unimac. Following sodium sulfite treatment the specimens were rinsed three times and then air dried flat before conducting felting shrinkage tests. To determine felting shrinkage, the differently-treated wool jersey fabrics were washed according to IWS TM31 (5x5A).

Figure 12 shows the FTIR/ATR spectra of wool jersey knit treated with (1) no enzyme (*i.e.*, PGDA+$H_2O_2$ only), (2) aryl esterase, (3) sodium sulfite, (4) no enzyme followed by sodium sulfite, and (4) aryl esterase followed by sodium sulfite. The appearance of a new peak at about 1023 $cm^{-1}$ was observed using specimens treated with sodium sulfite, indicating Bunte salt formation. As before, the signals at 1040 $cm^{-1}$ and 1170 $cm^{-1}$ are the result of arylesterase treatment.

As shown in the table in Figure 13, both arylesterase-treated and arylesterase + sodium sulfite-treated knit fabrics showed significantly reduced felting shrinkage compared to other treated fabric specimens. These results demonstrate that the combination of arylesterase-treatment and sodium sulfite treatment produce a fabric that exhibits even less shrinkage and softer hand compared to arylesterase-treatment alone.

## Claims

1. A method for reducing felting of wool or another textile made from keratinous fibers, the method comprising:

   contacting the textile with an aqueous composition comprising an enzyme having perhydrolase activity, an ester substrate for the enzyme, and a source of hydrogen peroxide,
   wherein the enzyme generates peracids *in situ* in an aqueous medium, and
   wherein the peracids modify the textile, thereby reducing the tendency of the textile to felt.

2. The method of claim 1, wherein the enzyme generates peracids in situ prior to contacting the textile with the aqueous composition.

3. The method of claim 1, wherein contacting the textile with the aqueous composition occurs prior to the enzyme generating peracids in situ.

4. The method of claim 1, wherein contacting the textile with the aqueous composition and the enzyme generating peracids in situ occur simultaneously.

5. The method of any of the preceding claims, wherein the textile is wool.

6. The method of any of the preceding claims, wherein the keratinous fibers are obtained from sheep.

7. The method of any of the preceding claims, wherein the enzyme having perhydrolase activity is a polypeptide having an amino acid sequence that is at least 70% identical to the amino acid sequence set forth in SEQ ID NO: 1 or SEQ ID NO: 2.

8. The method of any of the preceding claims, wherein the enzyme having perhydrolase activity is a polypeptide having an amino acid sequence that is at least 80% identical to the amino acid sequence set forth in SEQ ID NO: 1 or SEQ ID NO: 2.

9. The method of any of the preceding claims, wherein the enzyme having perhydrolase activity is a polypeptide having

an amino acid sequence that is at least 90% identical to the amino acid sequence set forth in SEQ ID NO: 1 or SEQ ID NO: 2.

**10.** The method of any of the preceding claims, wherein the enzyme having perhydrolase activity is derived from Mycobacterium smegmatis perhydrolase.

**11.** The method of any of the preceding claims, wherein the enzyme having perhydrolase activity is a variant of Mycobacterium smegmatis perhydrolase having the substitution S54V.

**12.** The method of any of the preceding claims, wherein ester substrate is propylene glycol diacetate (PGDA), triacetin, ethyl acetate, tributyrin, and/or ethylene glycol diacetate (EGDA).

**13.** The method of any of the preceding claims, wherein the source of hydrogen peroxide, is hydrogen peroxide, perborate, or percarbonate.

**14.** The method of any of the preceding claims, further comprising the step of:

treating the keratinous fiber or textiles comprising the keratinous fiber with sodium sulfite to further reduce felting of the textiles.

**15.** The method of any of the preceding claims, further comprising the step of:

treating the keratinous fiber or textiles comprising the keratinous fiber with transglutaminase to improve textile strength.

**Patentansprüche**

**1.** Verfahren zum Reduzieren des Verfilzens von Wolle oder einem anderen, aus keratinösen Fasern hergestellten Textilmaterial, wobei das Verfahren Folgendes umfasst:

das Kontaktieren des Textilmaterials mit einer wässrigen Zusammensetzung umfassend ein Enzym, das eine Perhydrolaseaktivität aufweist, ein Estersubstrat für das Enzym und eine Wasserstoffperoxidquelle,
wobei das Enzym Persäuren in situ in einem wässrigen Medium erzeugt und
wobei die Persäuren das Textilmaterial modifizieren, wodurch die Neigung des Textilmaterials zum Verfilzen reduziert wird.

**2.** Verfahren nach Anspruch 1, wobei das Enzym Persäuren in situ vor Kontaktieren des Textilmaterials mit der wässrigen Zusammensetzung erzeugt.

**3.** Verfahren nach Anspruch 1, wobei das Kontaktieren des Textilmaterials mit der wässrigen Zusammensetzung erfolgt, bevor das Enzym Persäuren in situ erzeugt.

**4.** Verfahren nach Anspruch 1, wobei das Kontaktieren des Textilmaterials mit der wässrigen Zusammensetzung und das Erzeugen von Persäuren in situ durch das Enzym gleichzeitig erfolgen.

**5.** Verfahren nach einem der vorhergehenden Ansprüche, wobei das Textilmaterial Wolle ist.

**6.** Verfahren nach einem der vorhergehenden Ansprüche, wobei die keratinösen Fasern von Schafen erhalten werden.

**7.** Verfahren nach einem der vorhergehenden Ansprüche, wobei das Enzym, das eine Perhydrolseaktivität aufweist, ein Polypeptid ist, das eine Aminosäuresequenz aufweist, die mindestens 70 % mit der Aminosäuresequenz identisch ist, die in SEQ ID NO: 1 oder SEQ ID NO: 2 aufgeführt ist.

**8.** Verfahren nach einem der vorhergehenden Ansprüche, wobei das Enzym, das eine Perhydrolaseaktivität aufweist, ein Polypeptid ist, das eine Aminosäuresequenz aufweist, die mindestens 80 % mit der Aminosäuresequenz identisch ist, die in SEQ ID NO: 1 oder SEQ ID NO: 2 aufgeführt ist.

**9.** Verfahren nach einem der vorhergehenden Ansprüche, wobei das Enzym, das eine Perhydrolaseaktivität aufweist, in Polypeptid ist, das eine Aminosäuresequenz aufweist, die mindestens 90 % mit der Aminosäuresequenz identisch ist, die in SEQ ID NO: 1 oder SEQ ID NO: 2 aufgeführt ist.

**10.** Verfahren nach einem der vorhergehenden Ansprüche, wobei das Enzym, das eine Perhydrolaseaktivität aufweist, von Mycobacterium-smegmatis-Perhydrolase abgeleitet ist.

**11.** Verfahren nach einem der vorhergehenden Ansprüche, wobei das Enzym, das eine Perhydrolaseaktivität aufweist, eine Variante von Mycobacterium-smegmatis-Perhydrolase ist, die die Substitution S54V aufweist.

**12.** Verfahren nach einem der vorhergehenden Ansprüche, wobei das Estersubstrat Propylenglycoldiacetat (PGDA), Triacetin, Ethylacetat, Tributyrin und/oder Ethylenglycoldiacetat (EGDA) ist.

**13.** Verfahren nach einem der vorhergehenden Ansprüche, wobei die Wasserstoffperoxidquelle Wasserstoffperoxid, Perborat oder Percarbonat ist.

**14.** Verfahren nach einem der vorhergehenden Ansprüche, des Weiteren den Schritt umfassend des:

Behandelns der keratinösen Faser oder der Textilmaterialien, die die keratinöse Faser umfassen, mit Natriumsulfit, um das Verfilzen der Textilmaterialien noch weiter zu reduzieren.

**15.** Verfahren nach einem der vorhergehenden Ansprüche, des Weiteren den Schritt umfassend des:

Behandelns der keratinösen Faser oder der Textilmaterialien, die die keratinöse Faser umfassen, mit Transglutaminase, um die Textilmaterialfestigkeit zu verbessern.

**Revendications**

**1.** Procédé de réduction du feutrage de la laine ou d'un autre textile fait à partir de fibres kératiniques, le procédé comprenant:

mettre le textile en contact avec une composition aqueuse comprenant une enzyme ayant une activité perhydrolase, un substrat ester pour l'enzyme et une source de peroxyde d'hydrogène,
où l'enzyme produit des peracides *in situ* dans un milieu aqueux, et
où les peracides modifient le textile, réduisant ainsi la tendance du textile à se feutrer.

**2.** Procédé selon la revendication 1, dans lequel l'enzyme produit des peracides *in situ* avant la mise en contact du textile avec la composition aqueuse.

**3.** Procédé selon la revendication 1, dans lequel la mise en contact du textile avec la composition aqueuse survient avant que l'enzyme ne produise des peracides *in situ.*

**4.** Procédé selon la revendication 1, dans lequel la mise en contact du textile avec la composition aqueuse et avec l'enzyme produisant des peracides *in situ* survient simultanément.

**5.** Procédé selon l'une quelconque des revendications précédentes, dans lequel le textile est de la laine.

**6.** Procédé selon l'une quelconque des revendications précédentes, dans lequel les fibres kératiniques sont obtenues du mouton.

**7.** Procédé selon l'une quelconque des revendications précédentes, dans lequel l'enzyme ayant une activité perhydrolase est un polypeptide ayant une séquence d'acides aminés qui est identique à au moins 70 % à la séquence d'acides aminés présentée dans la SEQ ID NO: 1 ou la SEQ ID NO: 2.

**8.** Procédé selon l'une quelconque des revendications précédentes, dans lequel l'enzyme ayant une activité perhydrolase est un polypeptide ayant une séquence d'acides aminés qui est identique à au moins 80 % à la séquence d'acides aminés présentée dans la SEQ ID NO: 1 ou la SEQ ID NO: 2.

**9.** Procédé selon l'une quelconque des revendications précédentes, dans lequel l'enzyme ayant une activité perhydrolase est un polypeptide ayant une séquence d'acides aminés qui est identique à au moins 90 % à la séquence d'acides aminés présentée dans la SEQ ID NO: 1 ou la SEQ ID NO: 2.

**10.** Procédé selon l'une quelconque des revendications précédentes, dans lequel l'enzyme ayant une activité perhydrolase est dérivée de perhydrolase de *Mycobacteriarm smegmatis.*

**11.** Procédé selon l'une quelconque des revendications précédentes, dans lequel l'enzyme ayant une activité perhydrolase est un variant de la perhydrolase de *Mycobacterium smegmatis* ayant la substitution S54V.

**12.** Procédé selon l'une quelconque des revendications précédentes, dans lequel le substrat ester est du diacétate de propylène glycol (PGDA), de la triacétine, de l'acétate d'éthyle, de la tributyrine et/ou du diacétate d'éthylène glycol (EGDA).

**13.** Procédé selon l'une quelconque des revendications précédentes, dans lequel la source de peroxyde d'hydrogène est du peroxyde d'hydrogène, du perborate ou du percarbonate.

**14.** Procédé selon l'une quelconque des revendications précédentes, comprenant en outre l'étape consistant à: traiter la fibre kératinique ou les textiles comprenant la fibre kératinique avec du sulfite de sodium afin de réduire encore plus le feutrage des textiles.

**15.** Procédé selon l'une quelconque des revendications précédentes, comprenant en outre l'étape consistant à: traiter la fibre kératinique ou les textiles comprenant la fibre kératinique avec de la transglutaminase pour améliorer la résistance des textiles.

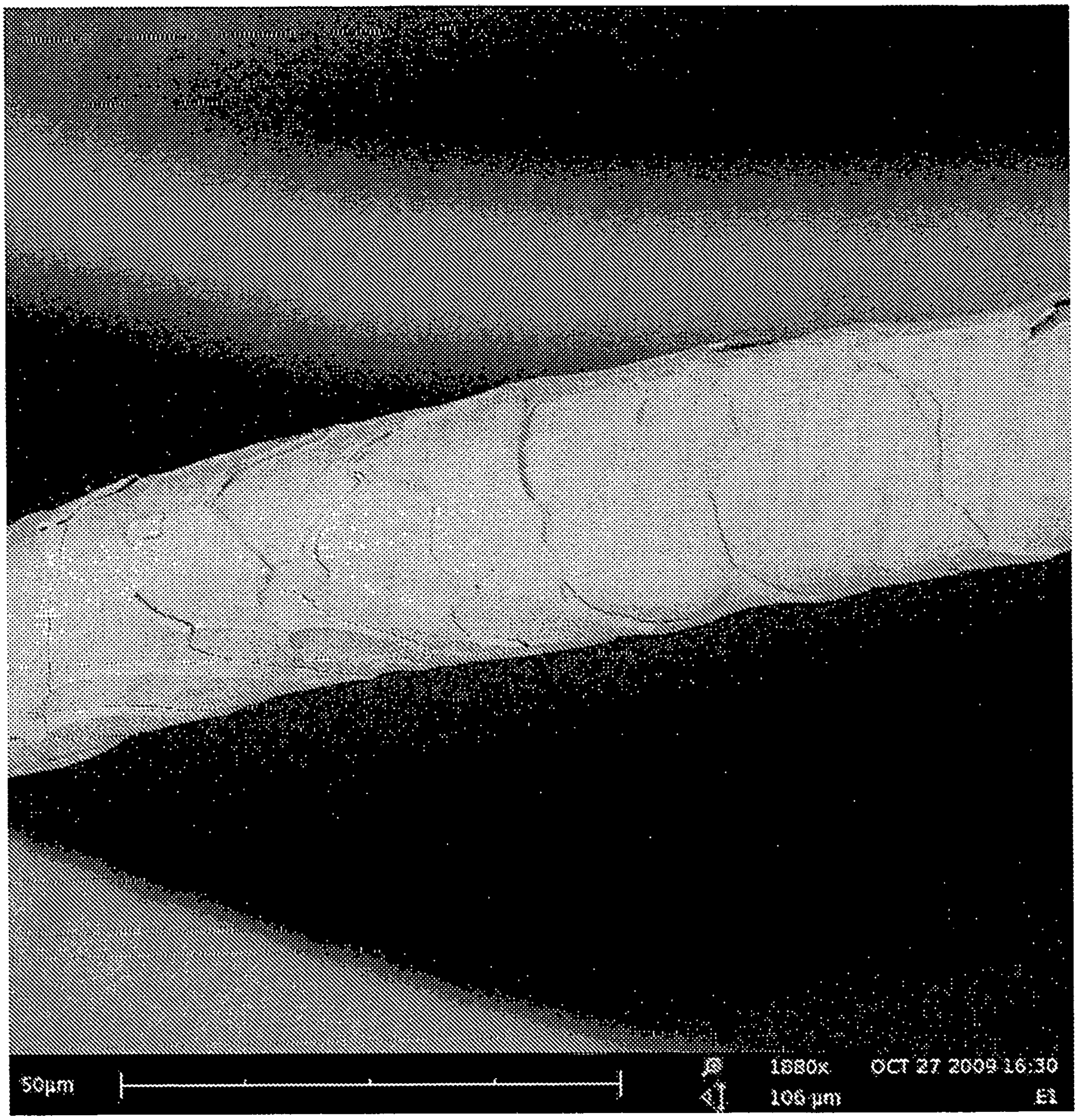


FIG. 1

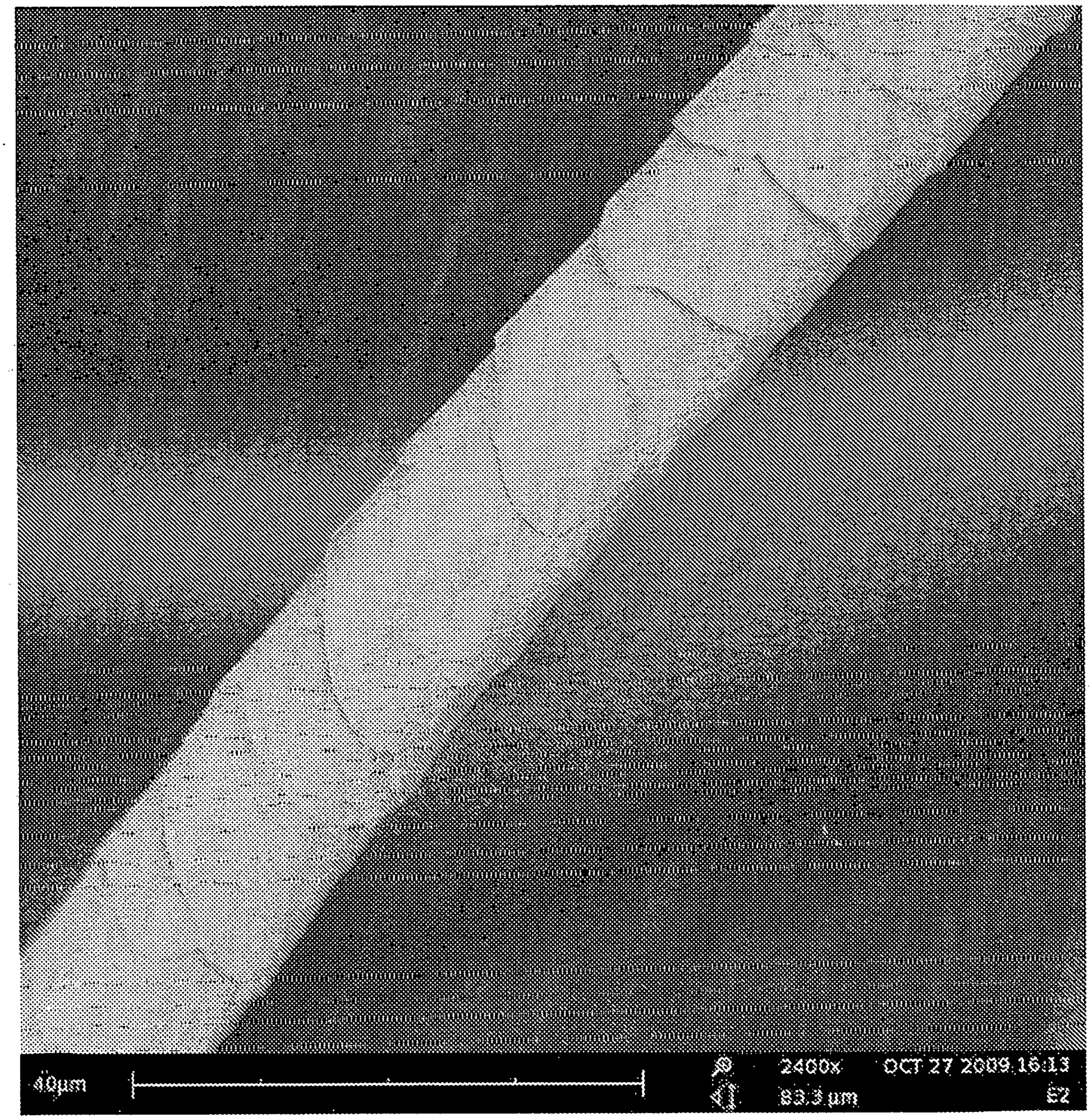


FIG. 2

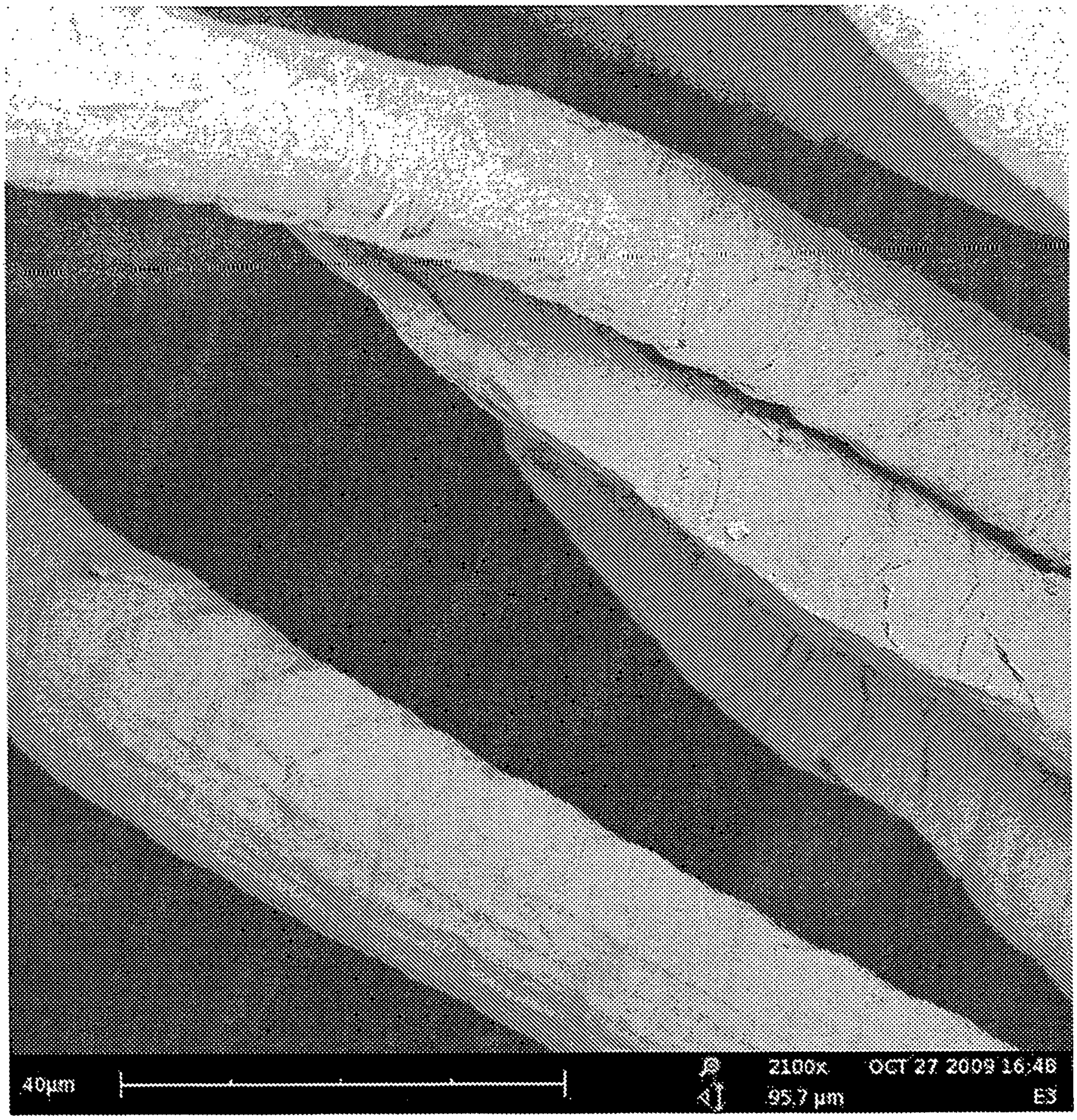


FIG. 3

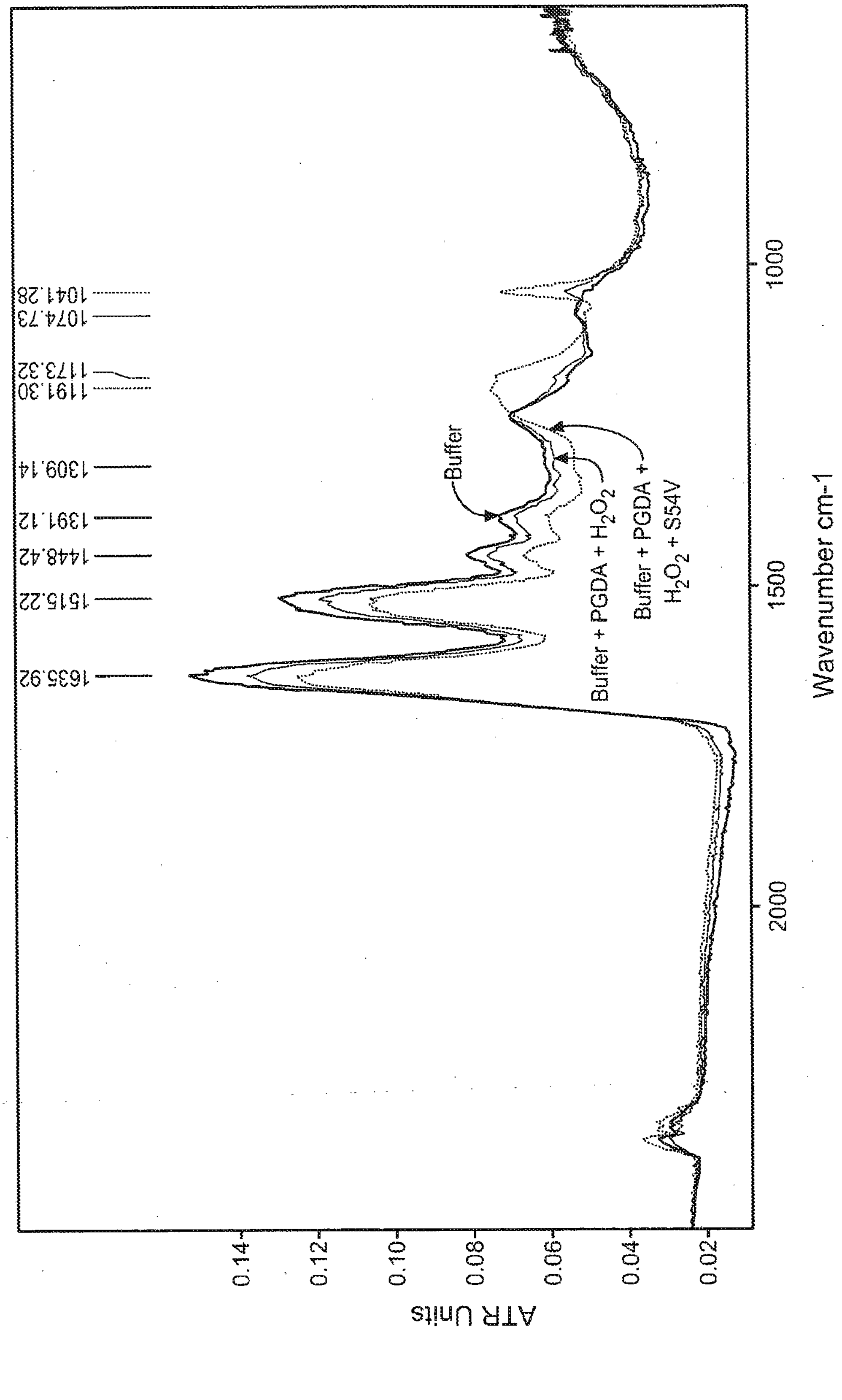
1635.92
1515.22
1448.42
1391.12
1309.14
1191.30
1173.32
1074.73
1041.28
Buffer
Buffer + PGDA + $H_2O_2$
Buffer + PGDA + $H_2O_2$ + S54V
2000
1500
1000
Wavenumber cm-1
0.14
0.12
0.10
0.08
0.06
0.04
0.02
ATR Units

*FIG. 4*

Control Dyed

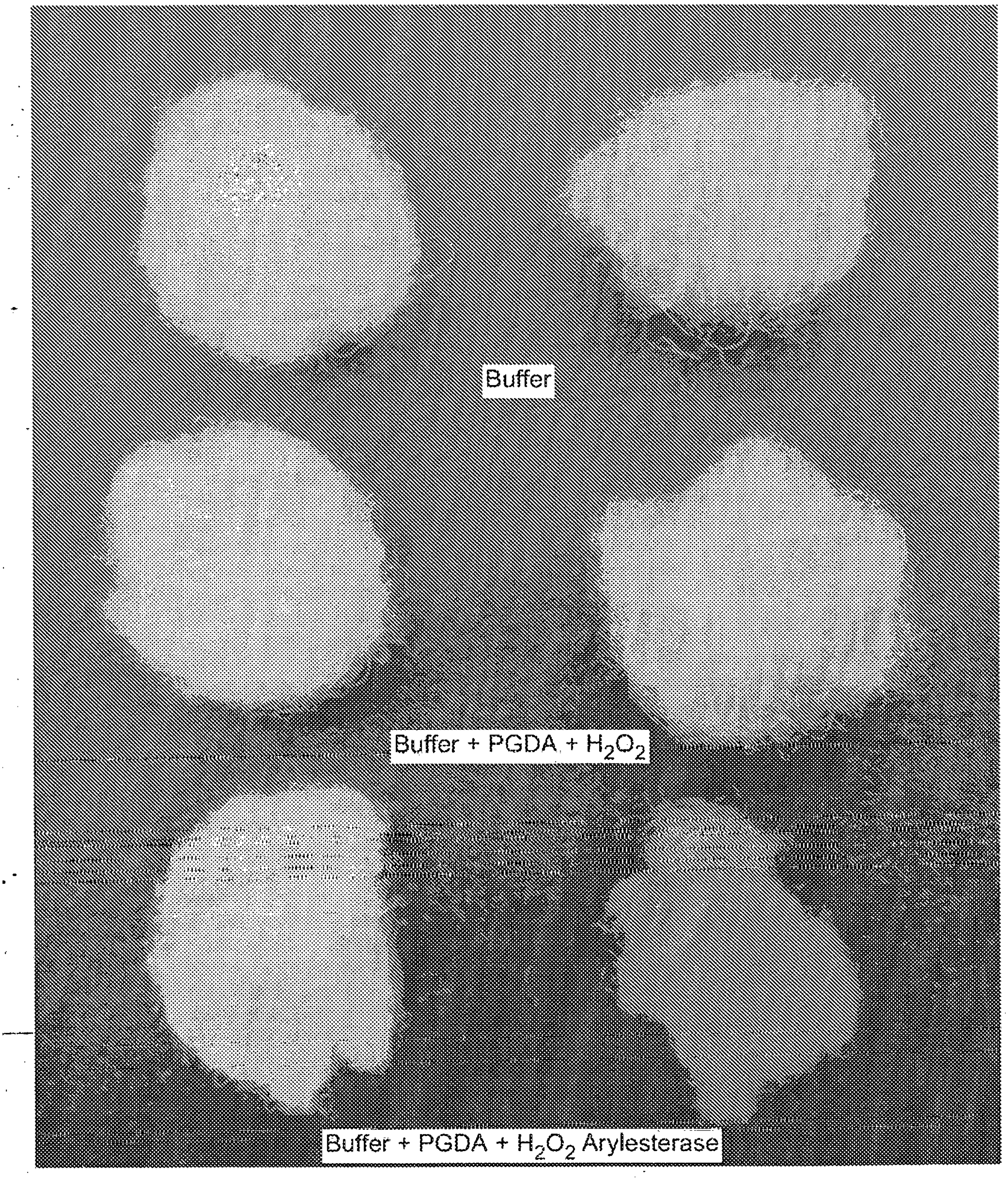


*FIG. 5*

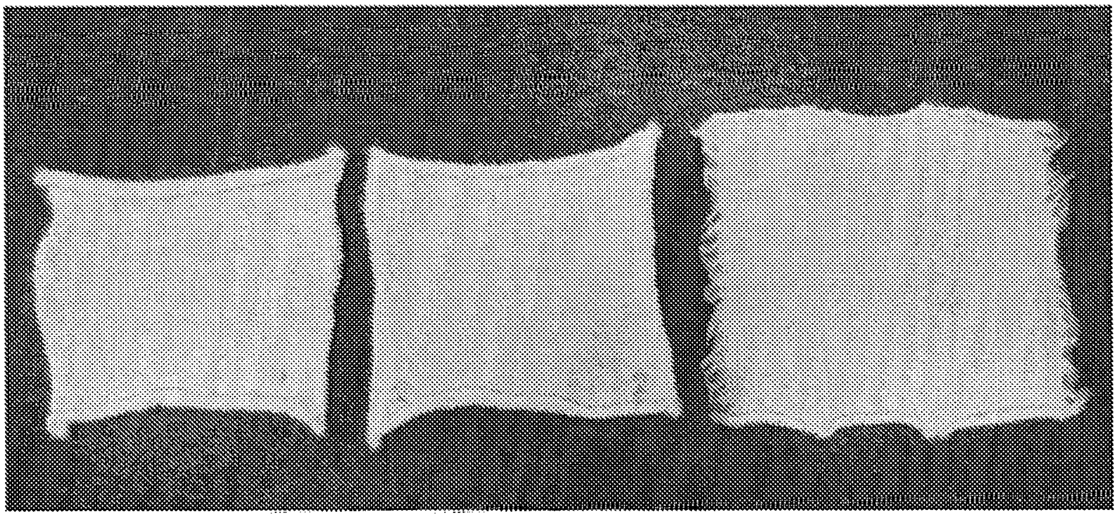

Buffer | Buffer + PGDA + $H_2O_2$ | Buffer + PGDA + $H_2O_2$ + Arylesterase

*FIG. 6*

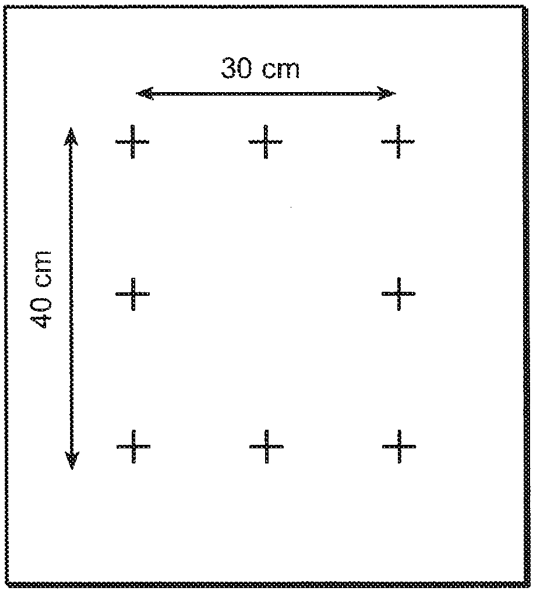


*FIG. 7*

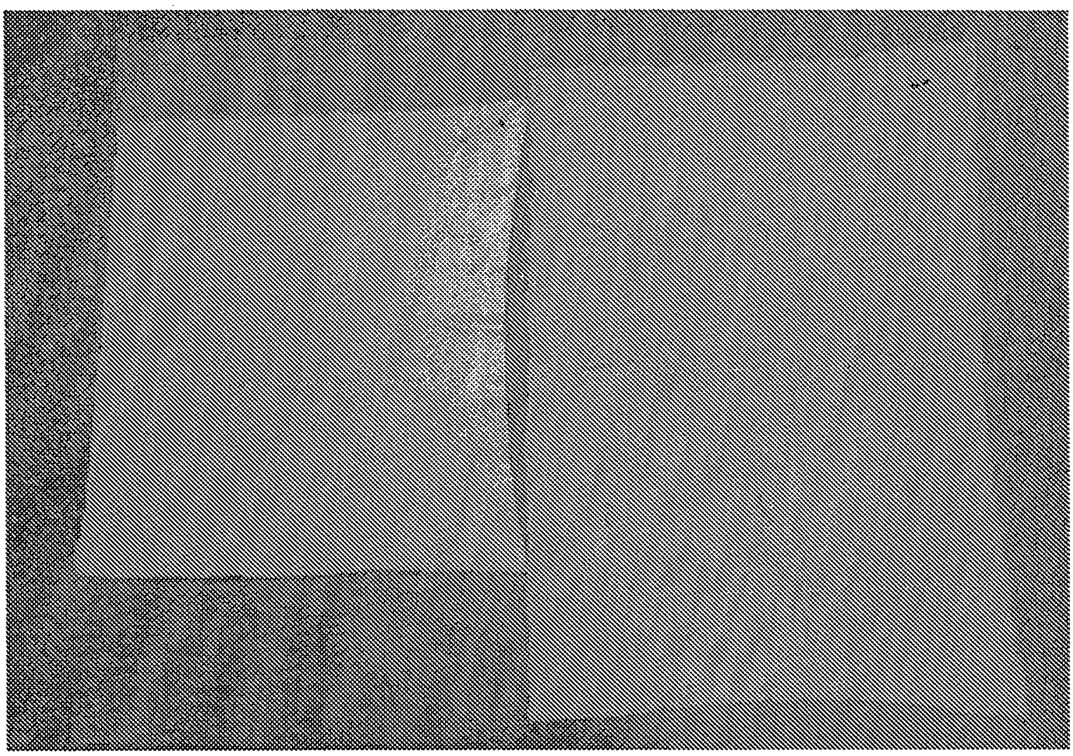

No Enzyme Control Arylesterase Treated

*FIG. 8*

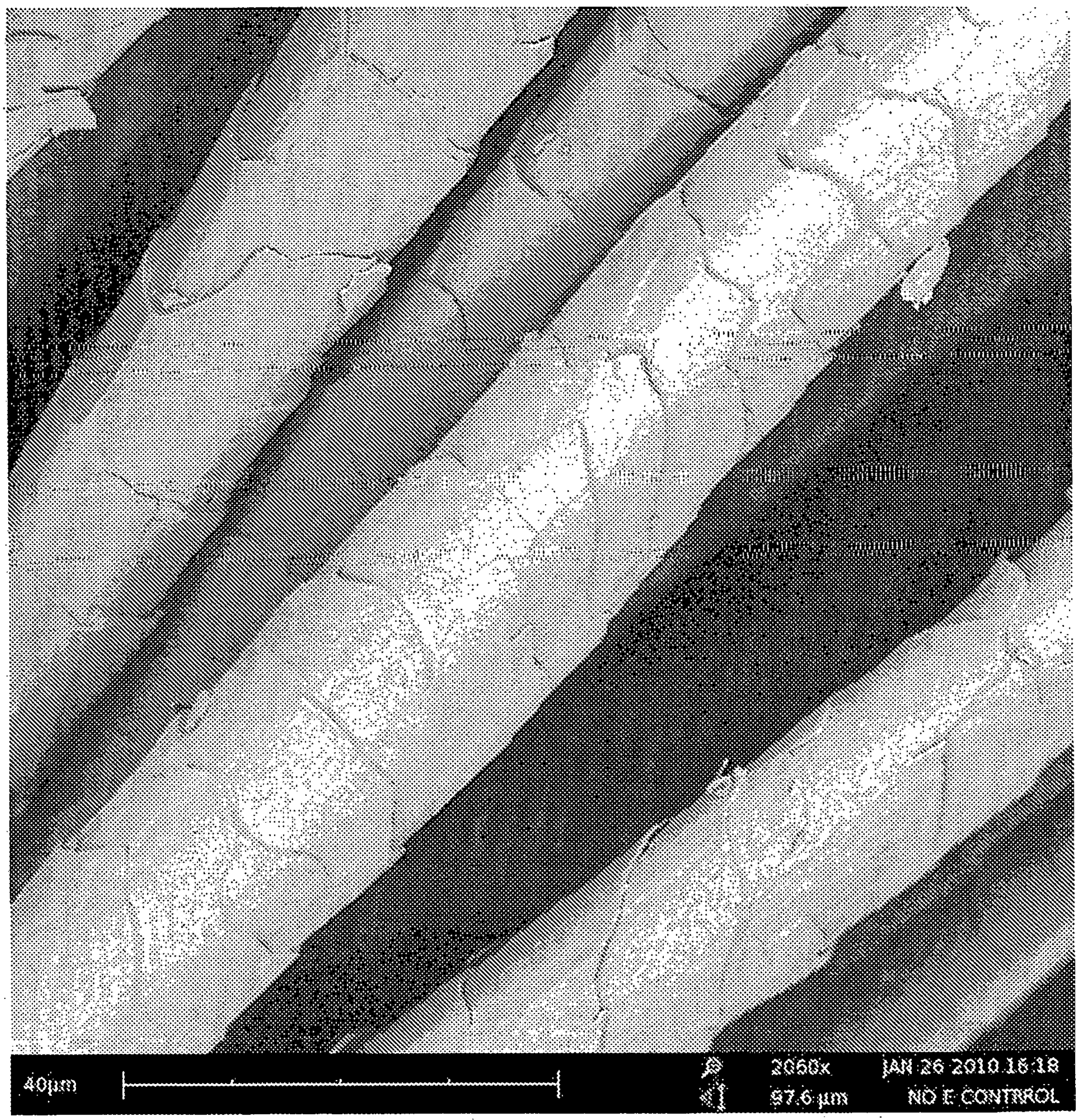


FIG. 9

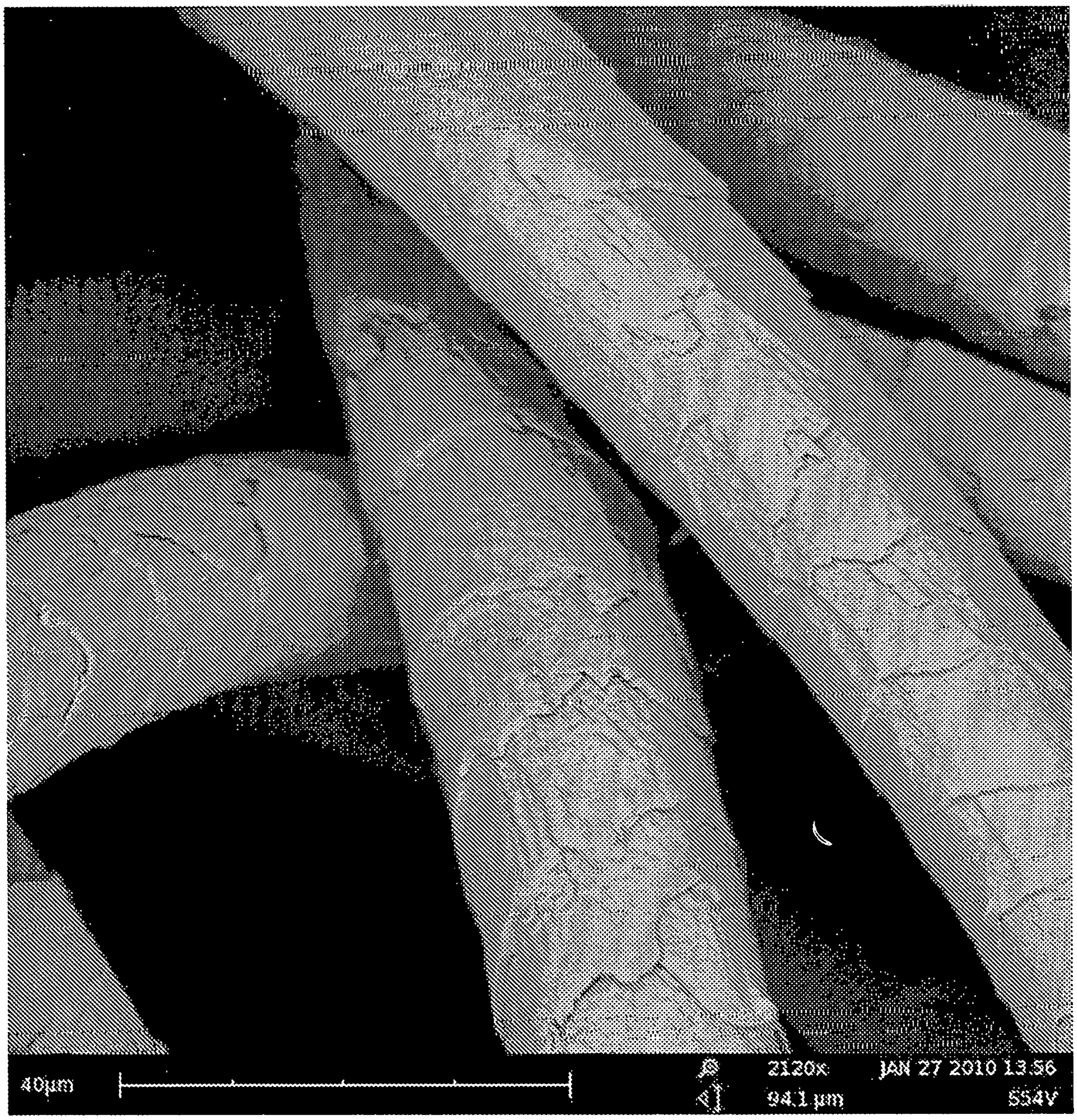


FIG. 10

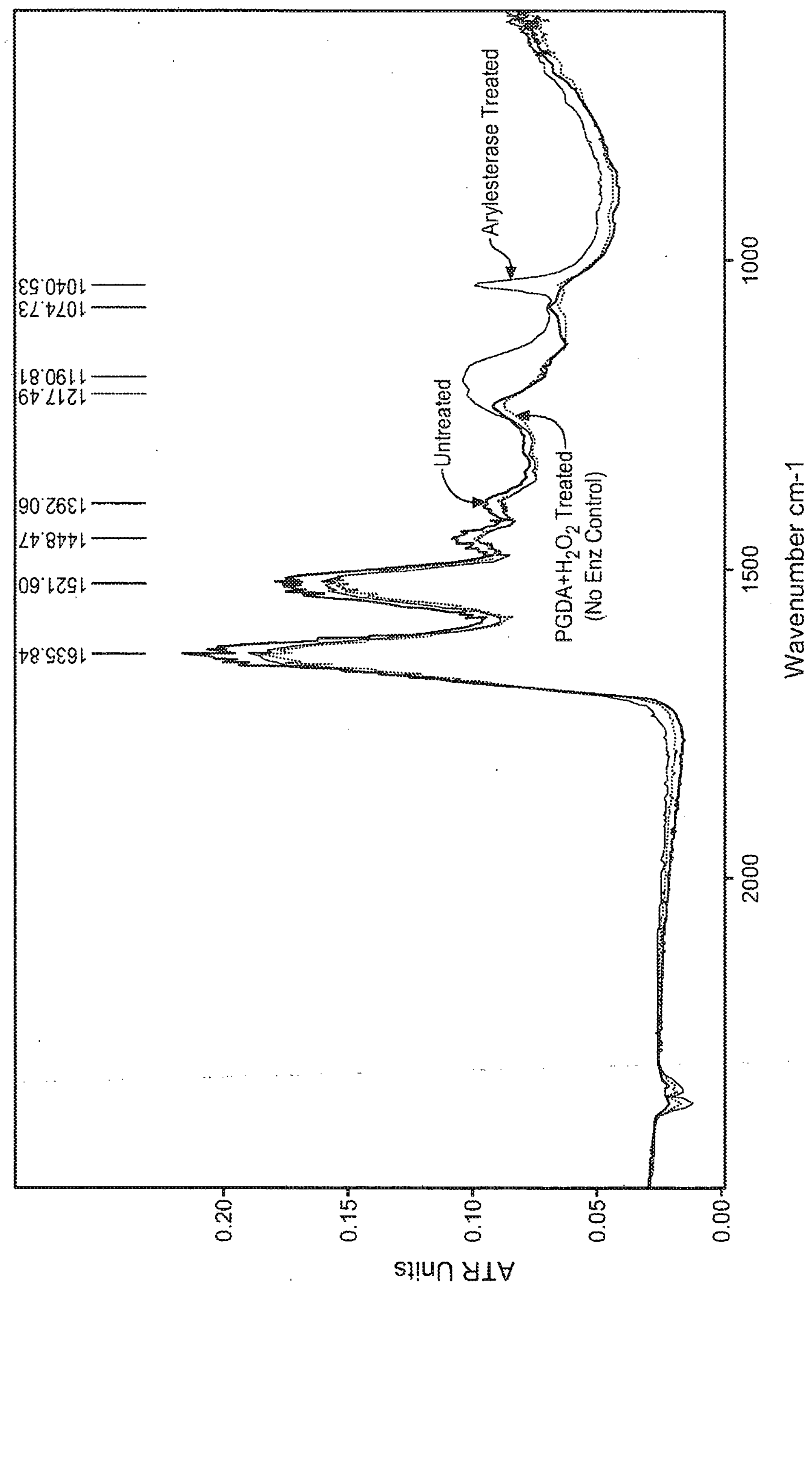
1635.84
1521.60
1448.47
1392.06
1217.49
1190.81
1074.73
1040.53
Arylesterase Treated
Untreated
PGDA+$H_2O_2$ Treated
(No Enz Control)
0.20
0.15
0.10
0.05
0.00
ATR Units
2000
1500
1000
Wavenumber cm-1

*FIG. 11*

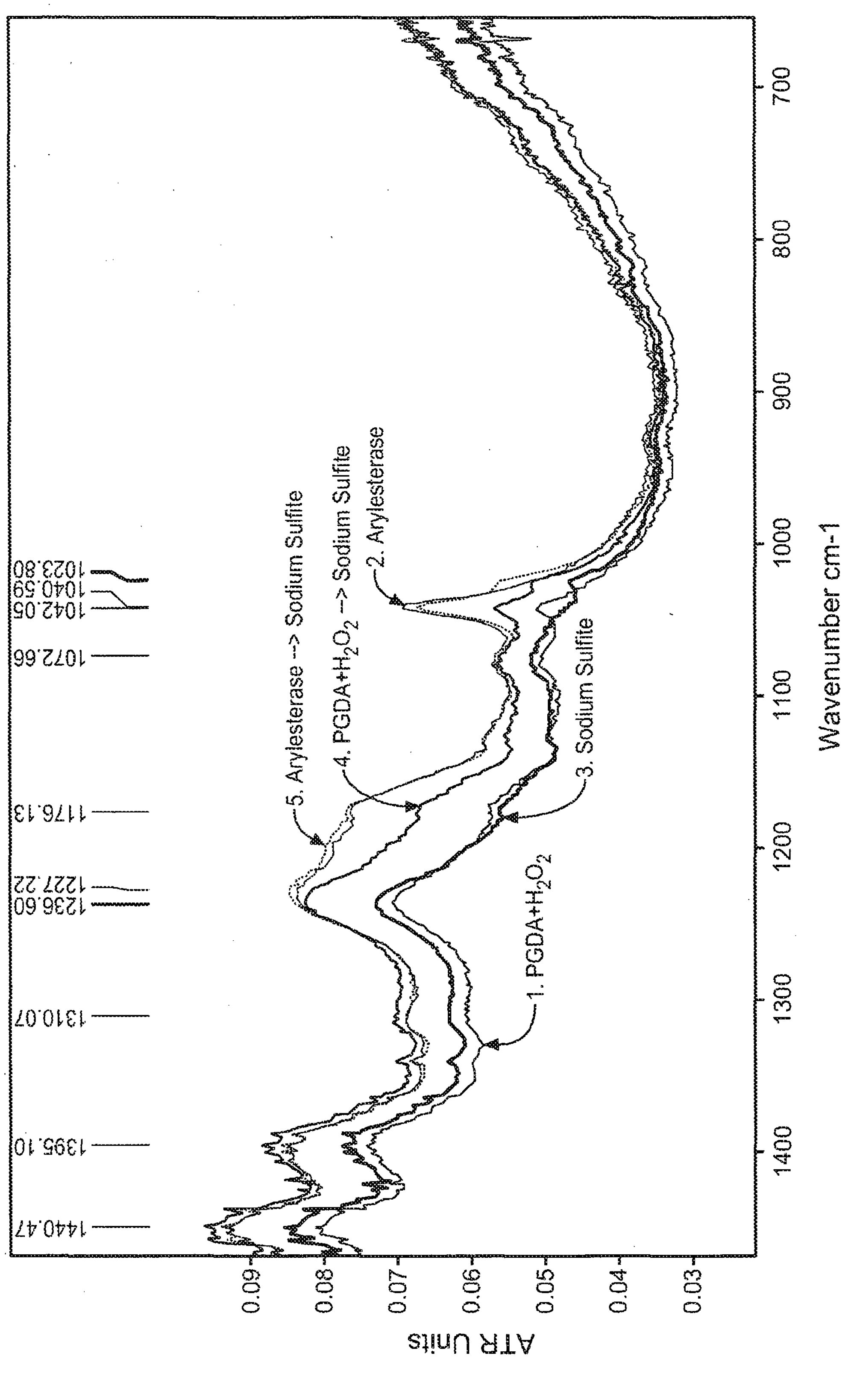
1440.47
1395.10
1310.07
1236.60
1227.22
1176.13
1072.66
1042.05
1040.59
1023.80
5. Arylesterase --> Sodium Sulfite
4. PGDA+$H_2O_2$ --> Sodium Sulfite
2. Arylesterase
3. Sodium Sulfite
1. PGDA+$H_2O_2$
ATR Units
0.09
0.08
0.07
0.06
0.05
0.04
0.03
1400
1300
1200
1100
1000
900
800
700
Wavenumber cm-1

**FIG. 12**

| Treatment | Direction | Measured Length in cm (% Shrinkage) | | | | |
|---|---|---|---|---|---|---|
| | | Original Length Before Treatment | Length After Treatment | Relaxation Shrinkage IWS TM 31 (1x7A) | Felting Shrinkage IWS TM 31 (5x5A) | Area Shrinkage |
| PGDA+$H_2O_2$ | Length (Machine) | 40.0 | 30.3 | 34.1 (14.8%) | 21.6 (36.5%) | (60.5%) |
| | Width | 29.7 | 21.8 | 25.6 (13.7%) | 19.5 (24.1%) | |
| Arylesterase Treated | Length (Machine) | 40.0 | 32.7 | 34.1 (14.8%) | 28.3 (17.1%) | (-22.4%) |
| | Width | 29.7 | 31.6 | 25.6 (13.7%) | 35.8 (-39.6%) | |

*FIG. 13*

| Treatment | Direction | Measured Length in cm (% Shrinkage) | | | | |
|---|---|---|---|---|---|---|
| | | Original Length Before Treatment | Length After Treatment | Relaxation Shrinkage IWS TM 31 (1x7A) | Felting Shrinkage IWS TM 31 (5x5A) | Area Shrinkage |
| PGDA+$H_2O_2$ | Length (Machine) | 40.0 | 37.2 | 38.1 (4.8%) | 29.7 (22.1%) | (30.6%) |
| | Width | 29.7 | 28.4 | 28.8 (3.2%) | 26.3 (8.5%) | |
| Arylesterase | Length (Machine) | 40.0 | 38.1 | 38.1 (4.8%) | 32.0 (16.0%) | (19.6%) |
| | Width | 29.7 | 28.4 | 28.8 (3.2%) | 27.7 (3.6%) | |
| Sodium Sulfite (SS) | Length (Machine) | 40.0 | 34.8 | 38.1 (4.8%) | 28.6 (25.0%) | (33.5%) |
| | Width | 29.7 | 29.0 | 28.8 (3.2%) | 26.3 (8.5%) | |
| PGDA+$H_2O_2$--> SS | Length (Machine) | 40.0 | 34.3 | 38.1 (4.8%) | 29.1 (23.7%) | (32.0%) |
| | Width | 29.7 | 28.5 | 28.8 (3.2%) | 26.4 (8.3%) | |
| Arylesterase --> SS | Length (Machine) | 40.0 | 37.1 | 38.1 (4.8%) | 32.6 (14.4%) | (15.4%) |
| | Width | 29.7 | 28.9 | 28.8 (3.2%) | 28.5 (1.0%) | |

*FIG. 14*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3634020 A
- WO 2010030769 A
- US 20070167344 A1
- US 20030154555 A1
- WO 05056782 A
- WO 08063400 A
- US 2008145353 A
- US 2007167344 A
- US 2011026613 W
- US 61317915 B


### Non-patent literature cited in the description


- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **DEVEREUX et al.** *Nucleic Acids Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *Proc. Natl. Acad. Sci. USA,* 1993, vol. 90, 5873-5787
- **ALTSCHUL et al.** *Meth. Enzymol.,* 1996, vol. 266, 460-480
- **HENIKOFF ; HENIKOFF.** *Proc. Natl. Acad. Sci. USA,* 1989, vol. 89, 10915
- **HENIKOFF et al.** *Proc. Natl. Acad. Sci. USA,* 1989, vol. 89, 10915
- **KARIN et al.** *Proc. Natl. Acad. Sci USA,* 1993, vol. 90, 5873
- **HIGGINS et al.** *Gene,* 1988, vol. 73, 237-244
- **PEARSON et al.** *Proc. Natl. Acad. Sci. USA,* 1988, vol. 85, 2444-2448
- **CORTEZ et al.** *Enzyme and Microbial Technology,* 2004, vol. 34, 64-72